# EUROPEAN PATENT SPECIFICATION

(11) **EP 1 173 224 B1**
(45) Date of publication and mention of the grant of the patent: **06.09.2006**
(21) Application number: 00923636.5
(22) Date of filing: 31.03.2000
(51) Int. Cl.: A61K 9/127, A61K 9/12, A61K 48/00

(54) **POLYETHYLENEIMINE:DNA FORMULATIONS FOR AEROSOL DELIVERY**
POLYETHYLENEIMINE: DNS-FORMULIERUNGEN FÜR DIE AEROSOL-VERABREICHUNG
POLYETHYLENEIMINE UTILISE DANS DES PREPARATIONS D'ADN ADMINISTREES PAR AEROSOL

(30) Priority: 02.04.1999 US 127575 P
(43) Date of publication of application: 23.01.2002
(73) Proprietor: RESEARCH DEVELOPMENT FOUNDATION, Carson City, Nevada 89703 (US)
(72) Inventor: DENSMORE, Charles, L., Jr., The Woodlands, TX 77381 (US); KNIGHT, Jack, Vernon, Houston, TX 77027 (US); WALDREP, John, Clifford, The Woodlands, TX 77381 (US); ORSON, Frank, McNair, Houston, TX 77005 (US); KINSEY, Berma, M., Houston, TX 77005 (US)
(74) Representative: Heaton, Joanne Marie
(86) International application number: PCT/US2000/040044
(87) International publication number: WO 2000/059548

(56) References cited:
- PILLAI, R. ET. AL.: 'Ultrasonic nebulization of cationic lipid-based gene delivery systems for airway administration' PHARMACEUTICAL RESEARCH vol. 15, no. 11, 1998, pages 1743 - 1747, XP002929741
- WIGHTMAN L. ET. AL.: 'Development of tranferrin-polycation/DNA based vectors for gene delivery to melanoma cells' JOURNAL OF DRUG TARGETING vol. 7, no. 4, 1999, pages 293 - 303, XP002929742
- OGRIS, M. ET. AL.: 'PEGylation DNA/transferrin-PEI complexes: reduced interaction with blood components, extended circulation in blood and potential for systemic gene delivery' GENE THERAPY 1999, pages 595 - 605, XP002929743
- BYRON, P. R. ET. AL.: 'Drug delivery via the respiratory tract' JOURNAL OF AEROSOL MEDICINE vol. 7, no. 1, 1994, pages 49 - 75, XP002929744
- KIRCHEIS, R. ET. AL.: 'Coupling of cell-binding ligands to polyethylenimine for targeted gene delivery' GENE THERAPY vol. 4, 1997, pages 409 - 418, XP002929745
- SCHIPPER, N. G. M. ET. AL.: 'The nasal mucociliary clearance: relevance to nasal drug delivery' PHARMACEUTICAL RESEARCH vol. 8, no. 7, 1991, pages 807 - 814, XP002929746
- MOREN. F.: 'Different techniques of aerosol administration to the lower respiratory tract' EUR. J. RESPIR. DIS. SUPPL. vol. 63, 1982, pages 15 - 18, XP002929747
- MATHIAS, N. R. ET. AL.: 'Targeted drug delivery to the respiratory tract: Solute permeability of air-interface cultured rabbit tracheal epithelial cell monolayers' JOURNAL OF DRUG TARGETING vol. 4, 1996, pages 79 - 86, XP002929748

## Description

### BACKGROUND OF THE INVENTION

### Field of the Invention

The present invention relates generally to the field of gene therapy and pharmaceutical drug delivery. More specifically, the present invention relates to aerosolized polyethyleneimine delivery of therapeutic genes.

### Description of the Related Art

A serious problem associated with the aerosol delivery of genes to the lungs (in the form of naked DNA, viruses or liposomes) is the marked decrease in efficiency that accompanies the process of nebulization compared to results obtained by *in vitro* transfection (Crook et al., 1996; Schwartz et al., 1996; Eastman et al., 1997a,b, 1998). In addition, pulmonary surfactant lipids and proteins have been shown to inhibit cationic liposome-mediated transfection (Duncan et al., 1997; Tsan et al., 1997). These effects probably contribute to the reported low *in vivo* gene transfer of lipid-based aerosolized formulations and have slowed the development of systems that will deliver DNA efficiently to the lungs.

Recently, it was reported that a formulation containing one particular cationic lipid (bis-guanidinium-tren-cholesterol; BGTC) was more stable during the process of nebulization than some previously tested lipids. Despite this improvement in *in vivo* transfection by aerosol, clinical applications of such therapy may require even more efficient delivery vectors. In recent years, polycations have been reported to be effective in transfecting cells *in vitro* and *in vivo.* One derivative with conspicuous activity is polyethyleneimine (PEI) (Boussif et al., 1995, 1996). It is herein reported that not only are PEI-DNA formulations resistant to nebulizer-induced reduction of transfection efficiency, but the formulation is much more efficient than lipid-based formulations in transfecting pulmonary tissues *in vivo,* such that polyethyleneimine-based formulations are about 10-fold more effective than previously optimized lipid-based formulations when delivered *in vivo* by jet nebulizer. Such polyethyleneimine-based formulations are more effective in transfecting the lungs, but produce relatively low levels of transfection in the nasal passages of mice. Polyethyleneimine-based formulations appear to be good candidates for aerosol delivery of genes for the treatment of a variety of genetic pulmonary disorders, including lung tumors, and, can be a noninvasive means of genetic immunization.

The prior art is deficient in non-lipid vehicles and formulations for aerosol delivery of DNA or therapeutic genes for use in targeted gene therapy via the respiratory tract. The present invention fulfills this long-standing need and desire in the art.

### SUMMARY OF THE INVENTION

Aerosol delivery of plasmid DNA to the lungs offers a noninvasive means of treating a variety of genetic pulmonary disorders. However, the process of nebulization frequently results in a rapid loss of transfection efficiency of many vector:DNA formulations. In conjunction with the design of aerosol delivery systems appropriate for DNA delivery, it is herein reported that formulations using polyethyleneimine and macromolecules such as DNA result in a high level of pulmonary transfection and are stable during nebulization. PEI-DNA formulations also exhibit a high degree of specificity for the lungs and are nontoxic. Optimal PEI-DNA ratios and concentrations have been determined for both *in vitro* and *in vivo* transfection. A serum factor appears to enhance PEI-mediated transfection, whereas surfactants have a minimal inhibitory effect.

One object of the present invention is to provide cationic, non-lipid vehicles and formulations to be used in aerosol delivery of DNA or therapeutic genes for targeted gene therapy via the respiratory tract.

In one embodiment of the present invention, there is provided a method of targeting gene therapy via the respiratory tract, comprising the step of: delivering aqueous dispersions via small particle aerosol of a therapeutic gene complexed with polyethyleneimine via the respiratory tract of an individual in need of such treatment.

In another embodiment of the present invention, there is provided a polycation-DNA composition for delivery of a therapeutic gene via small particle aerosol, comprising: polyethyleneimine; and DNA encoding a therapeutic gene.

In yet another embodiment of the present invention, there is provided a polyethyleneimine-DNA composition for delivery of a therapeutic gene via small particle aerosol, produced by a method comprising the steps of: dissolving DNA encoding a therapeutic gene in an appropriate solution; dissolving polyethyleneimine in an appropriate solution; complexing the dissolved DNA and the dissolved polyethyleneimine, producing a polyethyleneimine-DNA composition; and placing the polyethyleneimine-DNA composition in a device capable of nebulization, so as to produce a polyethyleneimine-DNA composition for delivery of a therapeutic gene via small particle aerosol.

Other and further aspects, features, and advantages of the present invention will be apparent from the following description of the presently preferred embodiments of the invention. These embodiments are given for the purpose of disclosure.

### BRIEF DESCRIPTION OF THE DRAWINGS

The appended drawings have been included herein so that the above-recited features, advantages and objects of the invention will become clear and can be understood in detail. These drawings form a part of the specification. It is to be noted, however, that the appended drawings illustrate preferred embodiments of the invention and should not be considered to limit the scope of the invention.

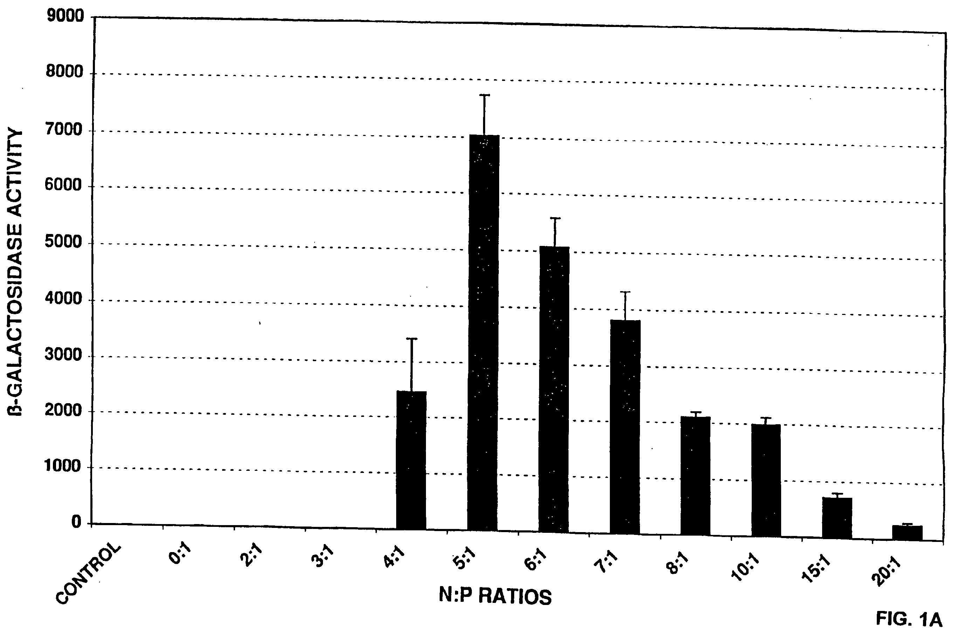

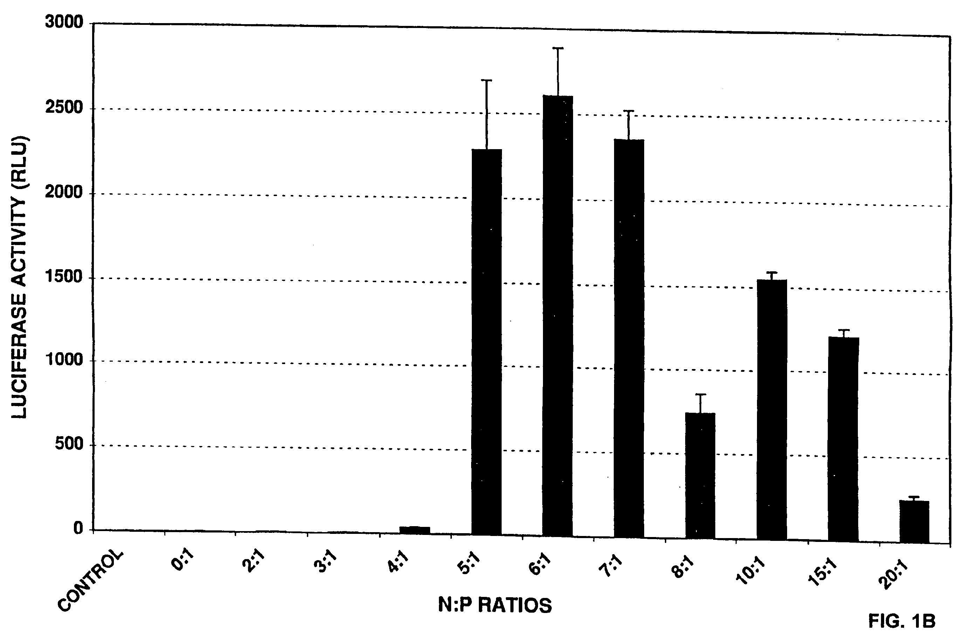

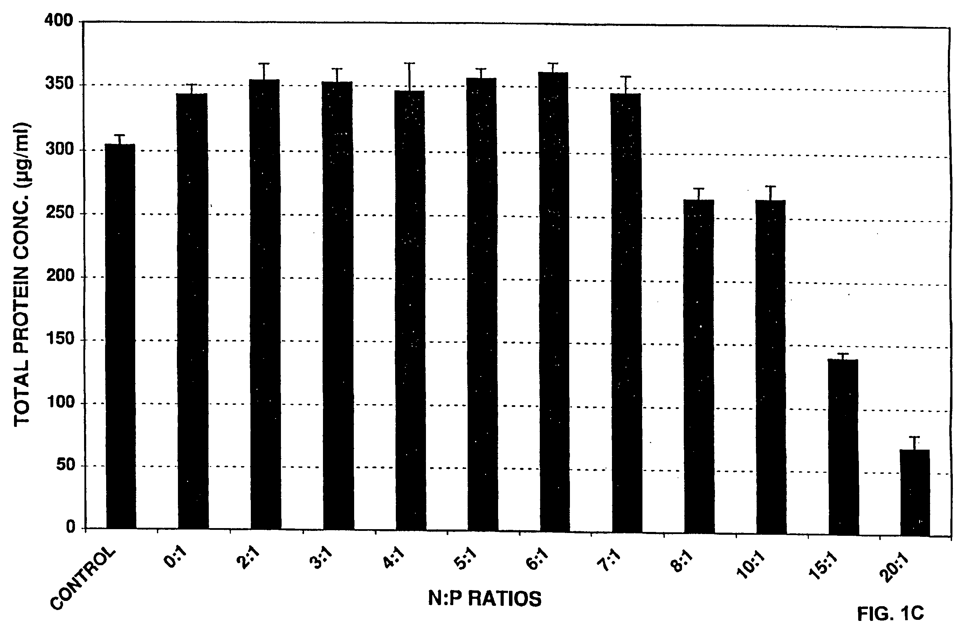

**Figure 1** shows transfection of A549 cells with PEI-DNA at various N:P ratios in the absence of serum. PEI-pCMVβ and PEI-luciferase formulations of increasing N:P ratio were prepared as described. A constant concentration of DNA was used throughout the experiment, except for "0" which was not transfected but which was exposed to otherwise identical media changes. A549 cells were transfected for 6 h and were extracted for β-galactosidase or luciferase activity 48 h after initiating transfection. **Figure 1A** illustrates the levels of β-galactosidase detected for each condition and **Figure 1B** illustrates the levels of luciferase determined for each condition. **Figure 1C** illustrates the levels of protein (an approximate measure of cell density and thus cytotoxicity) determined for cells transfected with PEI-pCMVβ (from the same experiment illustrated in Figure 1A). N = 3 for all groups. Bars indicate standard error.

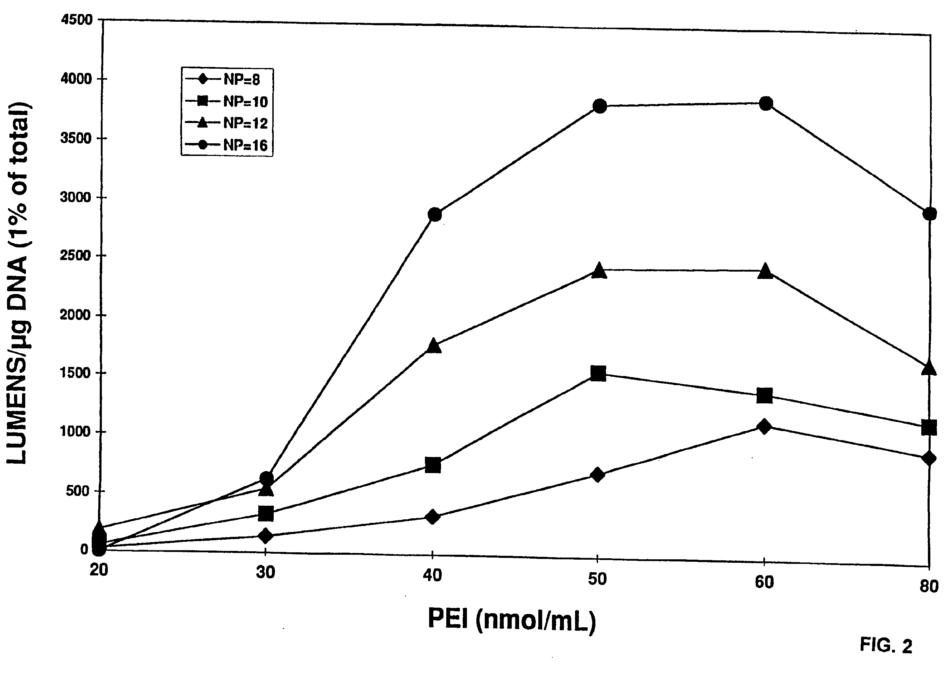

**Figure 2** shows transfection of A549 cells with increasing concentrations of PEI-DNA at different N:P ratios in the presence of serum. PEI-luciferase formulations of increasing concentration at the N:P ratios shown were prepared. A549 cells were transfected for 6 h and were extracted for luciferase activity 24 h after initiating transfection. N = 3 for all groups.

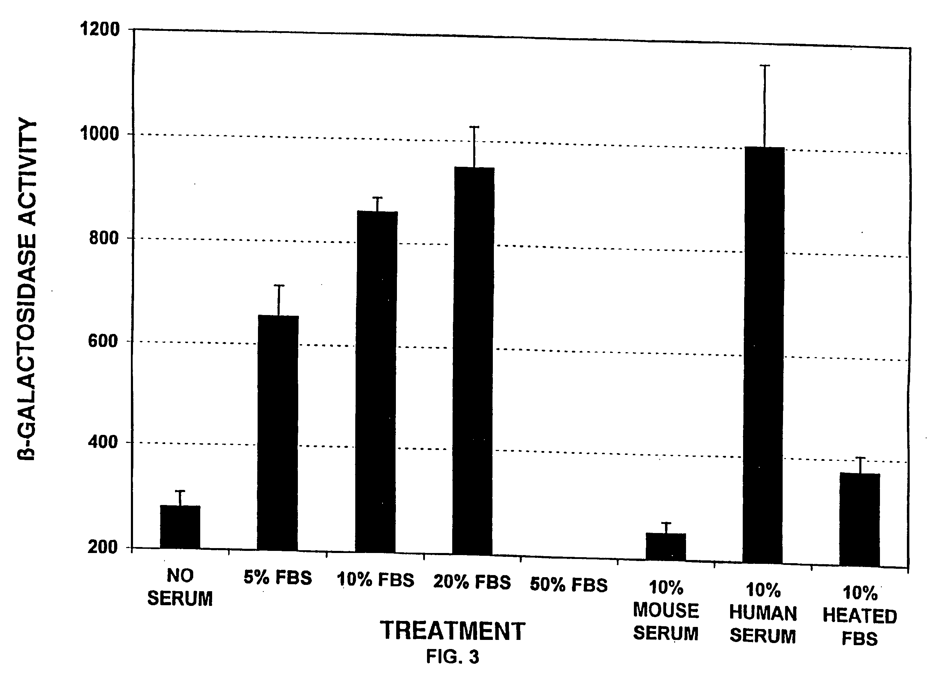

**Figure 3** shows the effects of serum on PEI-pCMVβ transfection efficiency *in vitro.* A549 cells were transfected with a constant concentration of DNA (1 µg/well) and PEI (15 nmol/well) at a constant N:P ratio of 20 with the additions indicated for 6 hours and were extracted for β-galactosidase 48 hours after initiating transfection. N = 3 for all groups. Bars indicate standard error.

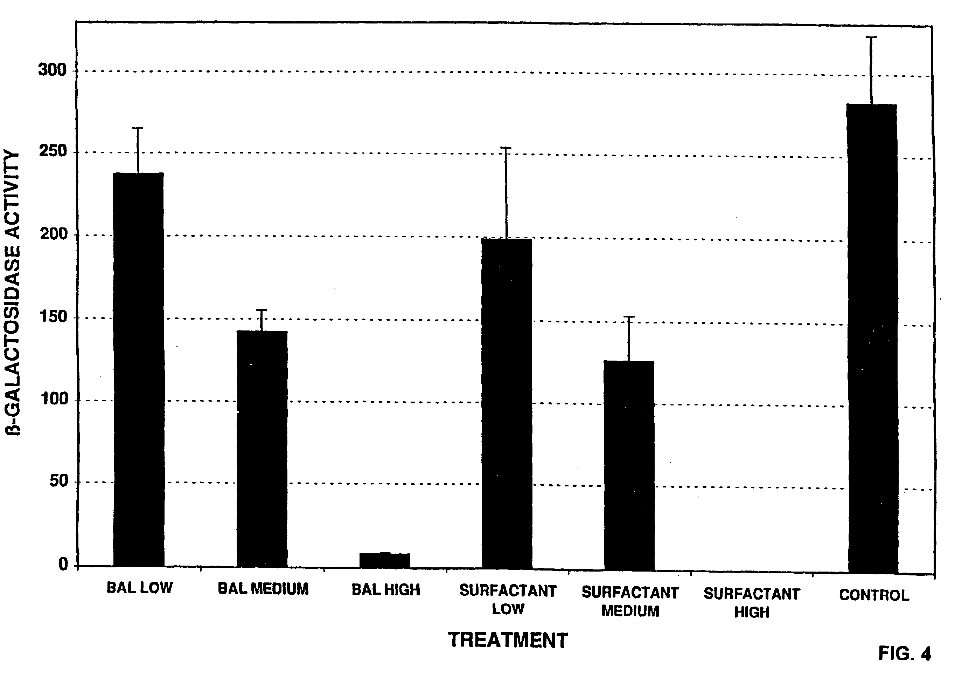

**Figure 4** shows the effects of surfactant and BAL fluid on PEI-pCMVβ transfection efficiency *in vitro.* A549 cells were transfected with a constant concentration of DNA (1 µg/well) and polyethyleneimine (15 nmol/well) at a constant N:P ratio of 20 with the additions incubated for 6 h and were then examined for β-galactosidase 48 h after initiating transfection. N = 3 for all groups. Bars indicate standard error.

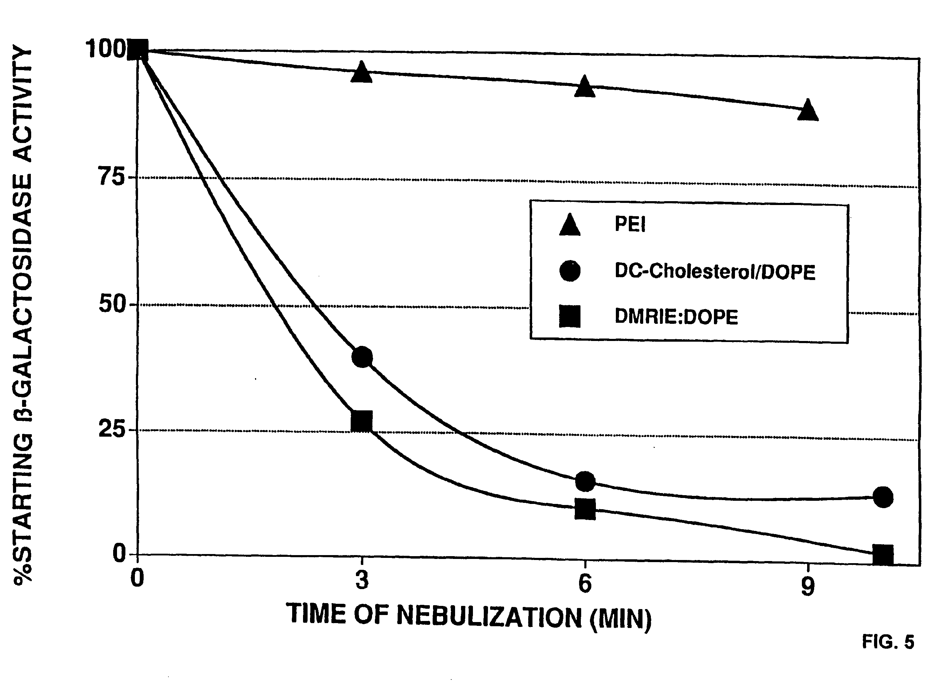

**Figure 5** shows the effect of polyethyleneimine and cationic lipid on transfection stability during nebulization. The PEI-pCMVβ and lipid-pCMVβ formulations were nebulized for the time periods shown prior to samples being taken from the nebulizer reservoir and qualitatively monitored in an *in vitro* transfection (CPRG) assay using A549 cells in culture as described. N = 3 for all groups.

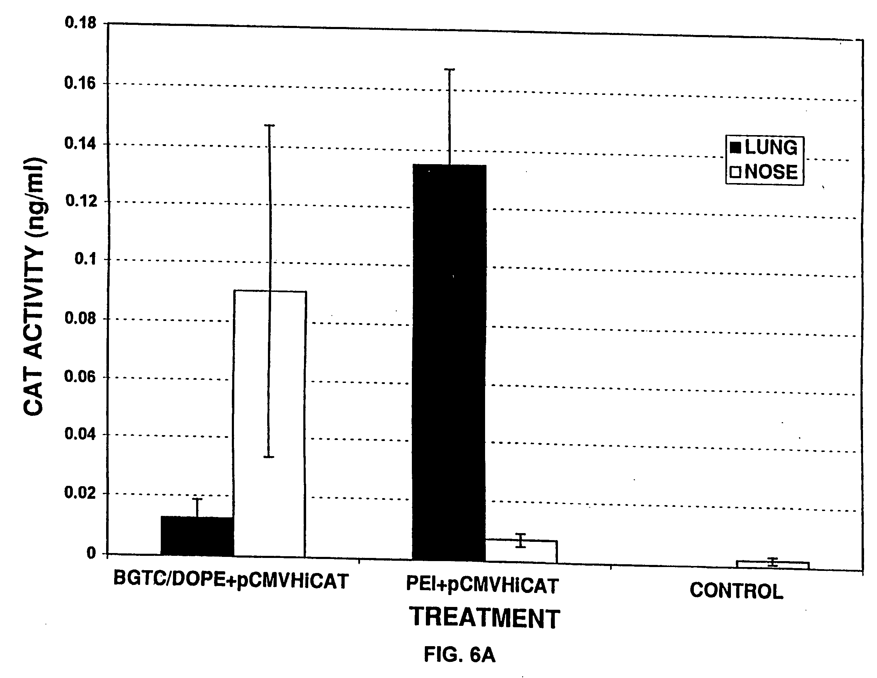

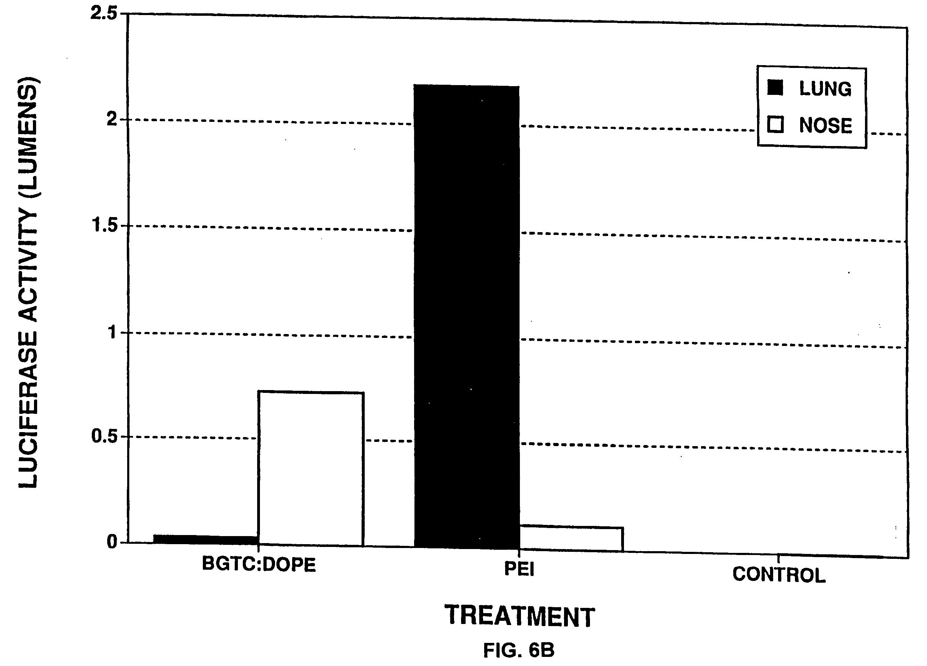

**Figure 6** shows *in vitro* intranasal treatment of Balb/c mice. In this experiment, mice were given an intranasal administration of the pCMVHi-CAT plasmid (containing the CAT gene) formulated with either polyethyleneimine or the cationic lipid BGTC:DOPE (**Figure 6A**). Equal amounts of DNA (24 µg) were given to all animals. Animals were first anesthetized and the DNA-vector solution was administered intranasally. The animals were sacrificed 48 h after instillation, lungs were removed and tissues were extracted and assayed (by ELISA) for the expression of CAT activity. An identical experiment was performed using a luciferase expression plasmid (pGL3) formulated with either polyethyleneimine or BGTC:DOPE (**Figure 6B**). Animals were sacrificed 48 hours after instillation, lungs and nose tissues were removed and extracted and assayed for luciferase expression as indicated. Each treatment group consisted of 6 animals. Bars indicate standard error.

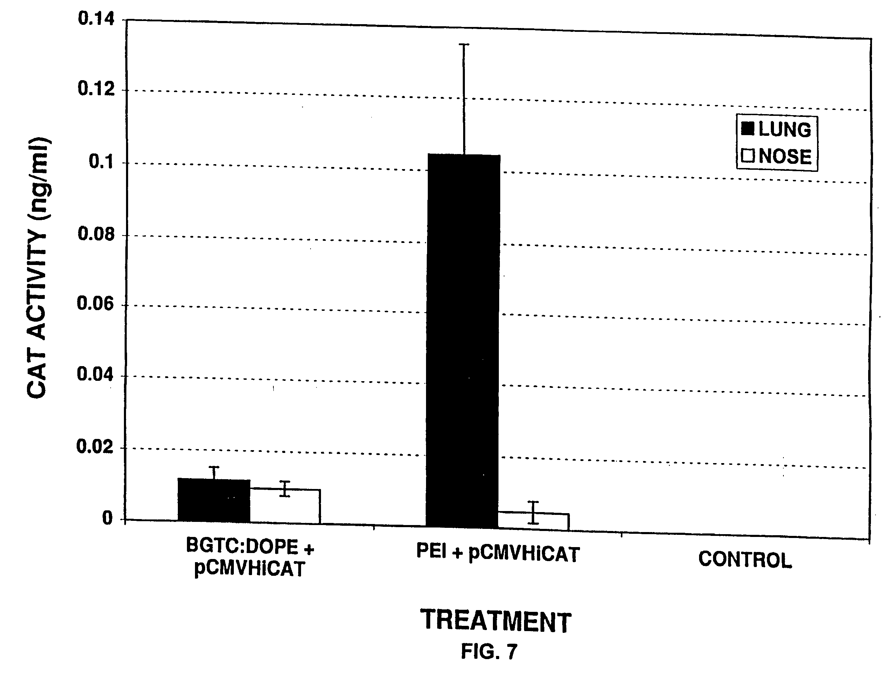

**Figure 7** shows *in vivo* aerosol treatment of Balb/c mice. In this experiment, animals were given either intermittent aerosol exposure of the PEI-DNA (chloramphenicol acetyl transferase gene), lipid-DNA (same plasmid, same DNA concentration) or no aerosol exposure (control). The exposure was accomplished by placing the animals in a sealed chamber attached to a Puritan Bennett 1600 single jet nebulizer and providing 1 min of aerosol treatment followed by a 9 min delay to allow the animals to breath the aerosol before beginning the cycle again. This was repeated until all of the nebulizer fluid (40 ml total) was depleted (approximately 16 h). Animals were sacrificed 48 h after the end of the aerosol treatment, lungs were removed and tissues were extracted and assayed (by ELISA) for the expression of CAT activity. Each treatment group consisted of 6 animals. Bars indicate standard error.

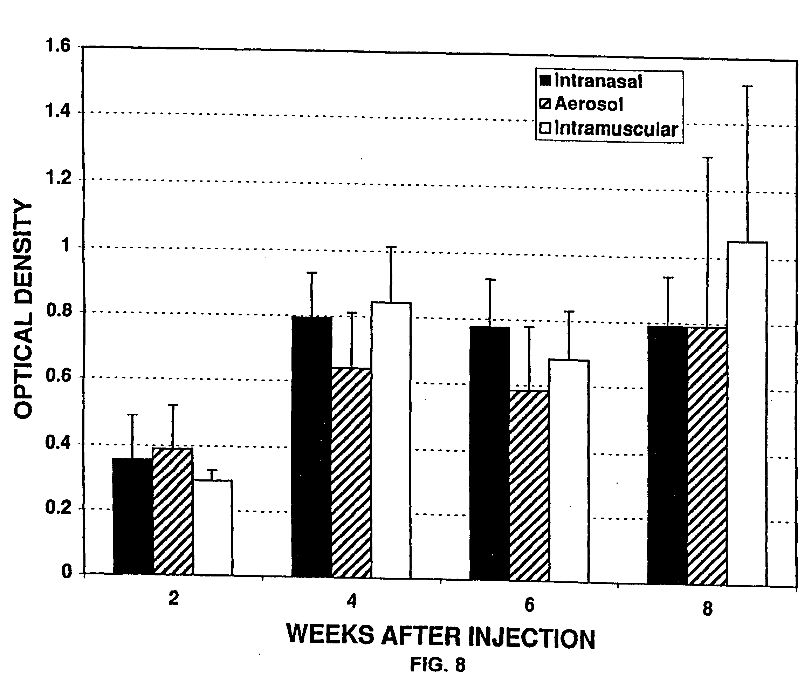

**Figure 8** shows antibody induction by genetic immunization with PEI-pCMV-hGH. Groups of 5 mice were exposed to the PEI-pCMV-hGH preparation by aerosol or intranasal instillation and the results were compared to those obtained with naked DNA given by intramuscular injection. Serum samples were obtained at 2 week intervals, and the presence of anti-hGH antibodies was measured by ELISA. Mean optical densities and standard deviation for each group is shown at a dilution of 1:500. The background OD of sera from unimmunized mice was 0.1 in this assay. Bars represent standard deviation.

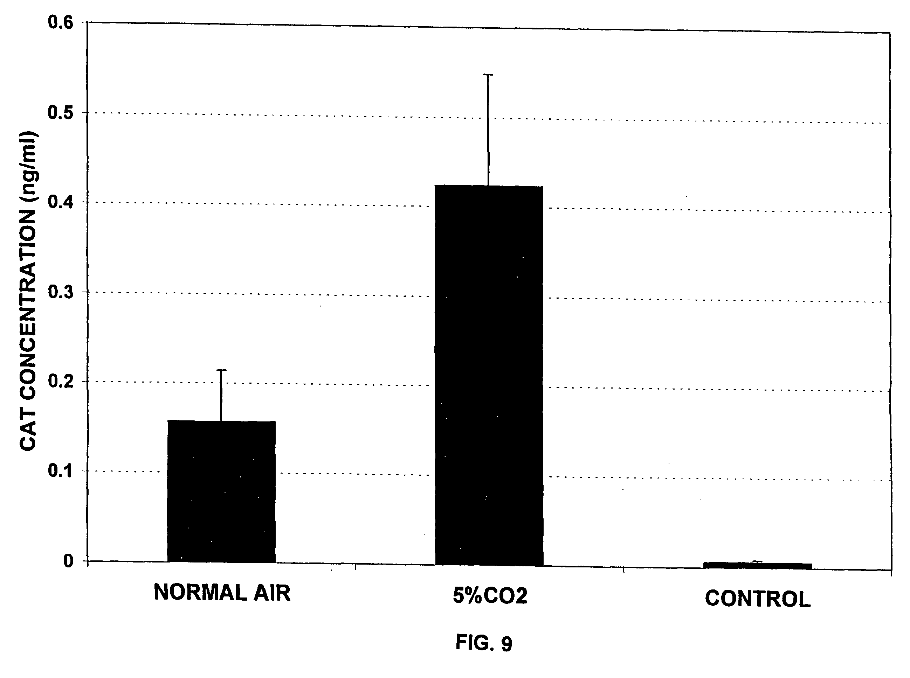

**Figure 9** shows a comparison between CAT expression in lung by PEI-DNA aerosol generated using air or air containing 5% CO₂. 1 mg of CAT plasmid was complexed with **PEI** at a N:P ratio of 10:1 and the resulting complex aerosolized to mice for 30 minutes. The lungs were harvested after 24 hours and the CAT assay performed as described. Values are mean ± SD (n = 6 mice per group, p = 0.001).

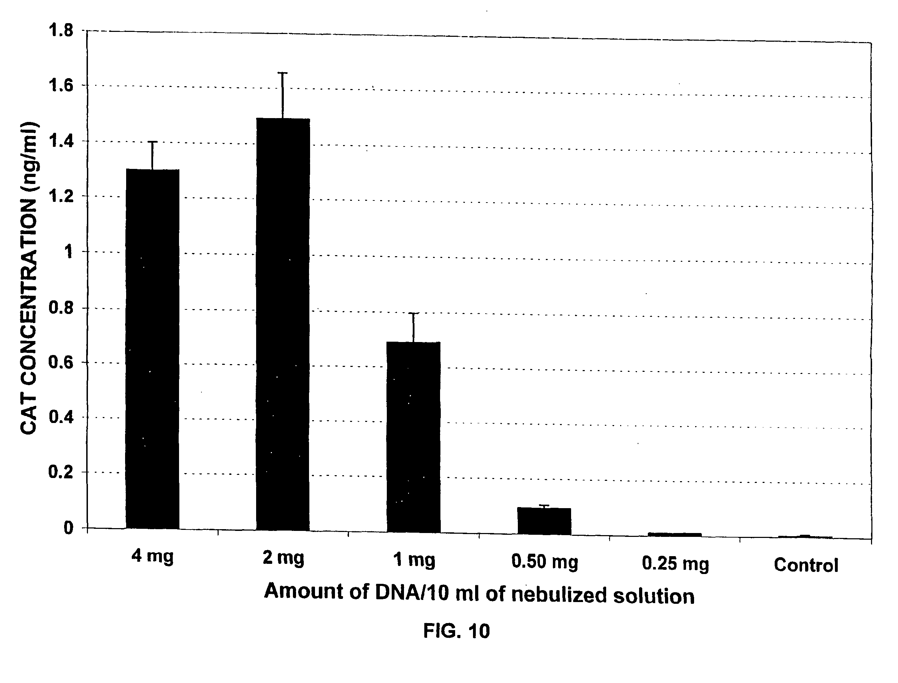

**Figure 10** shows that gene expression in lung by PEI-DNA aerosol is dose dependent. Increasing doses of CAT plasmid were aerosolized using 5% CO₂-in-air at a fixed N:P ratio of 10:1. There is an increase in both the total amount of DNA delivered and the concentration of PEI-DNA delivered. Mice were sacrificed after 24 hour, the lungs were harvested and the CAT protein was assayed. Values are mean ± SD (n = 5 mice per group).

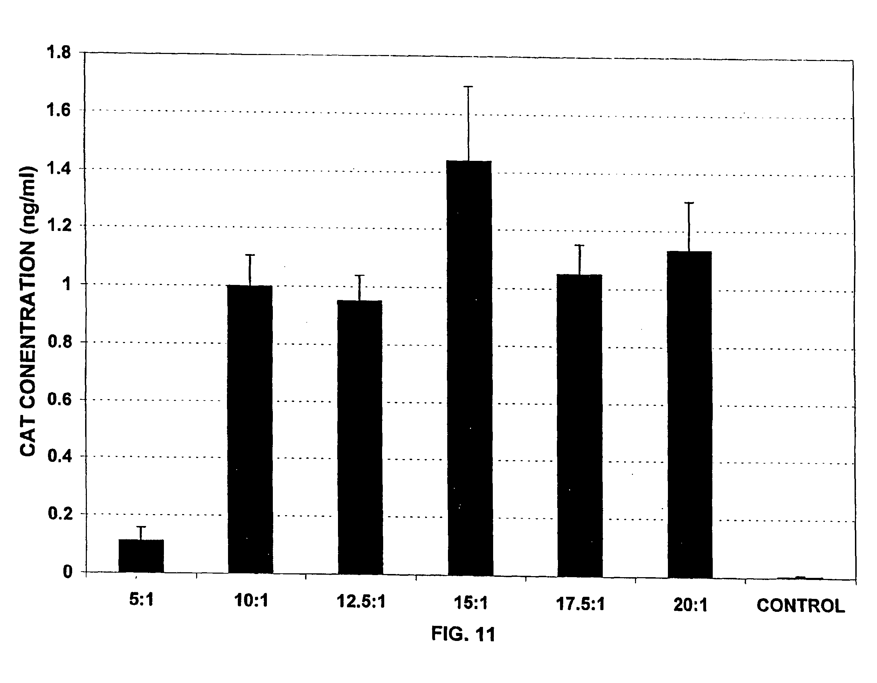

**Figure 11** shows the effect of N:P ratios on the efficiency of PEI-DNA transfer to the lung by aerosol. Different PEI-DNA (N:P) ratios were used with a fixed amount of CAT plasmid (2 mg). The complex was aerosolized using 5% CO₂-in-air. Mice were sacrificed after 24 hour, the lungs harvested and the CAT assay was performed. Values are mean ± SD (n = 5 mice per group).

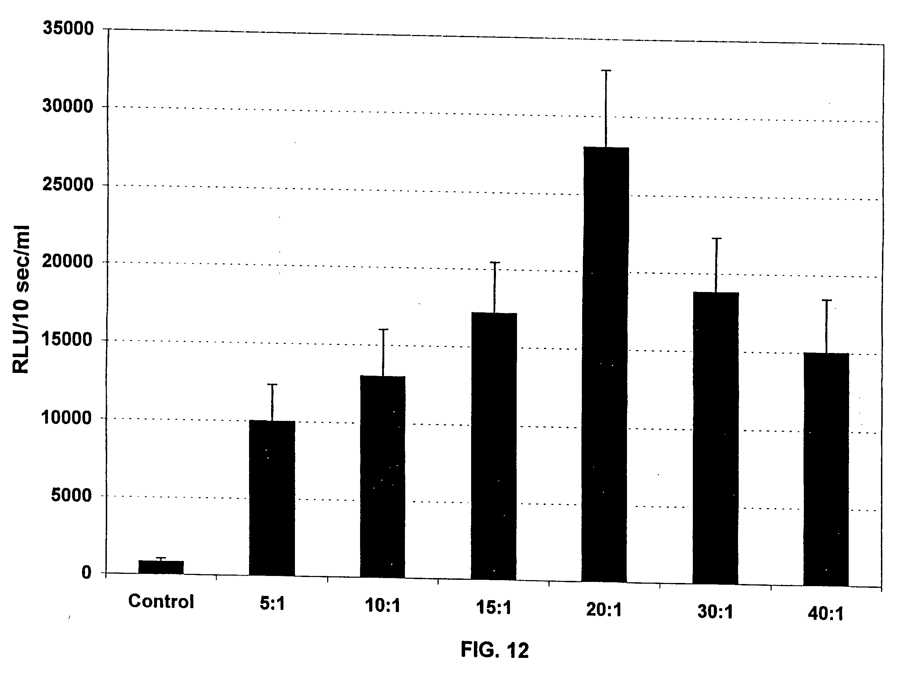

**Figure 12** shows the effect of N:P ratios on luciferase gene expression in the lung. A fixed amount of luciferase plasmid (2 mg) was delivered at different N:P ratios. The complexes were aerosolized using 5% CO₂-in-air. Mice were sacrificed 24 hour after aerosol delivery, lungs harvested and luciferase activity was determined. Values are mean ± SD (n = 5 mice per group).

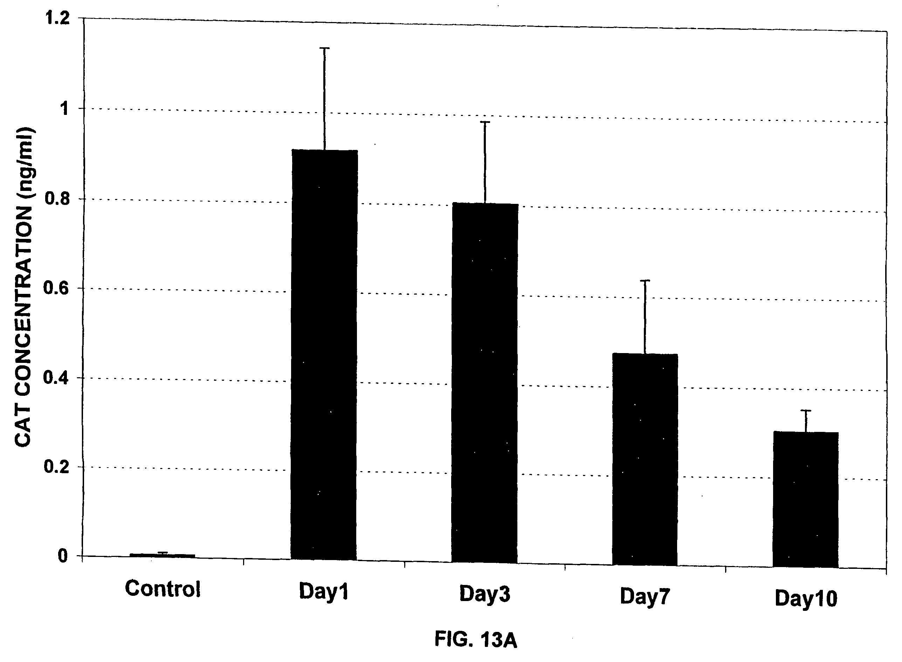

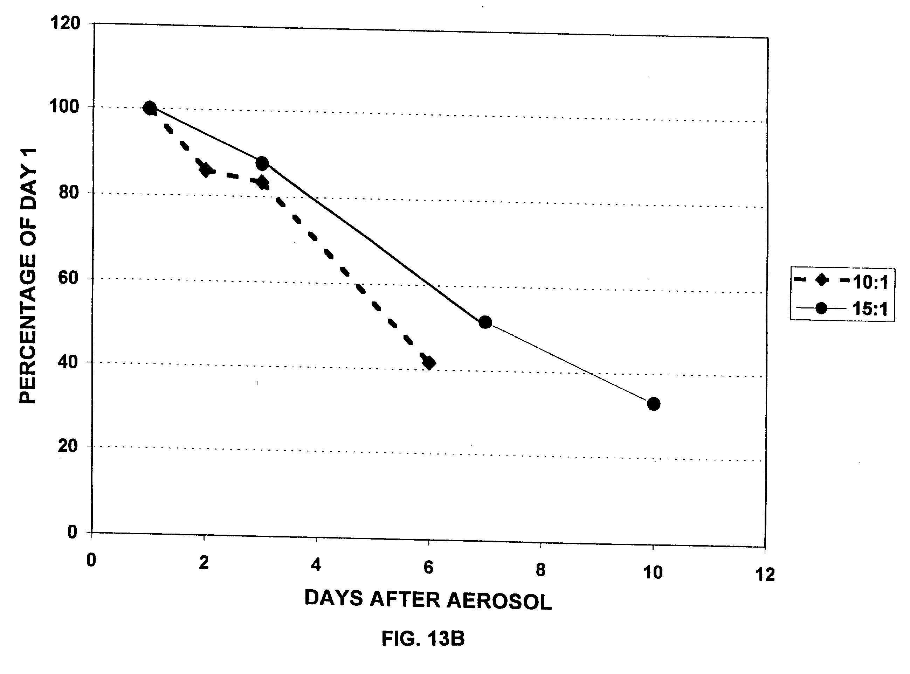

**Figure 13** shows the time course of transgene expression after single PEI-DNA aerosol exposure. **Figure 13A:** Mice were aerosolized with 2 mg of CAT plasmid at a N:P ratio of 15:1 using 5% CO₂-in-air. Mice were sacrificed at different time points and the lungs were harvested and immediately frozen. The CAT assay was performed after the last time point. Values are mean ± SD (n = 5 mice per time point). **Figure 13B**: Persistence of CAT expression using two different N:P ratios. Both groups of mice (n = 5 mice each time point per group) were delivered 2 m g of CAT plasmid at 15:1 or 10:1 N:P ratio using 5% CO₂-in-air. The time points for 10:1 ratio are 1,2,3 and 6 days post aerosol exposure and for 15:1 are 1,3,7, and 10 days post aerosol exposure.

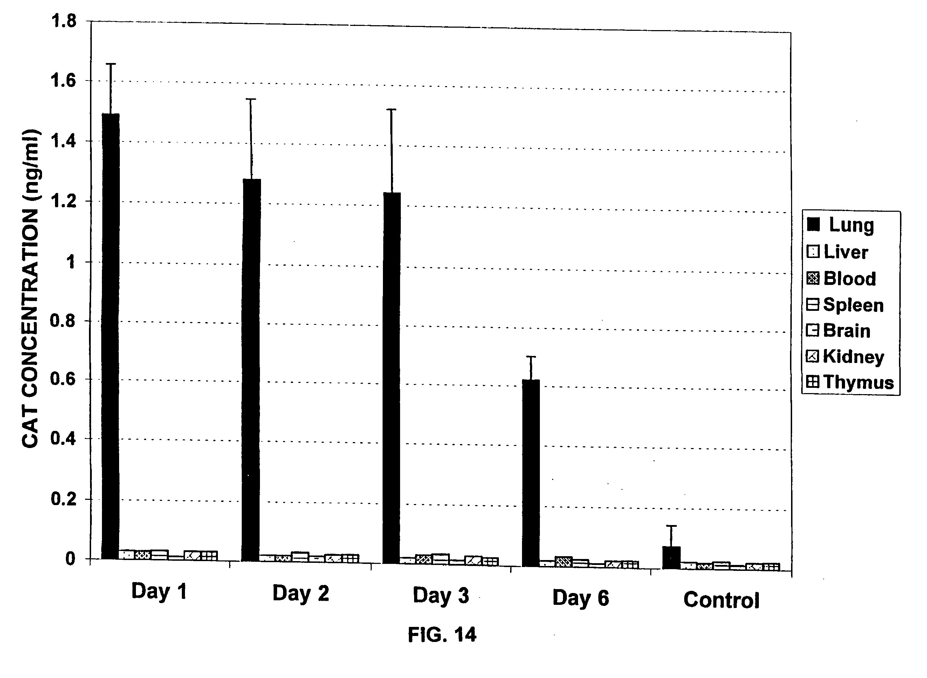

**Figure 14** shows a tissue distribution of transgene after single PEI-DNA aerosol exposure. The same groups of mice were used as in Figure 13 (from 10:1 group). Different tissues were harvested and immediately frozen. The CAT protein was assayed after the last time point. Values are mean ± SD (n = 5 mice per time point).

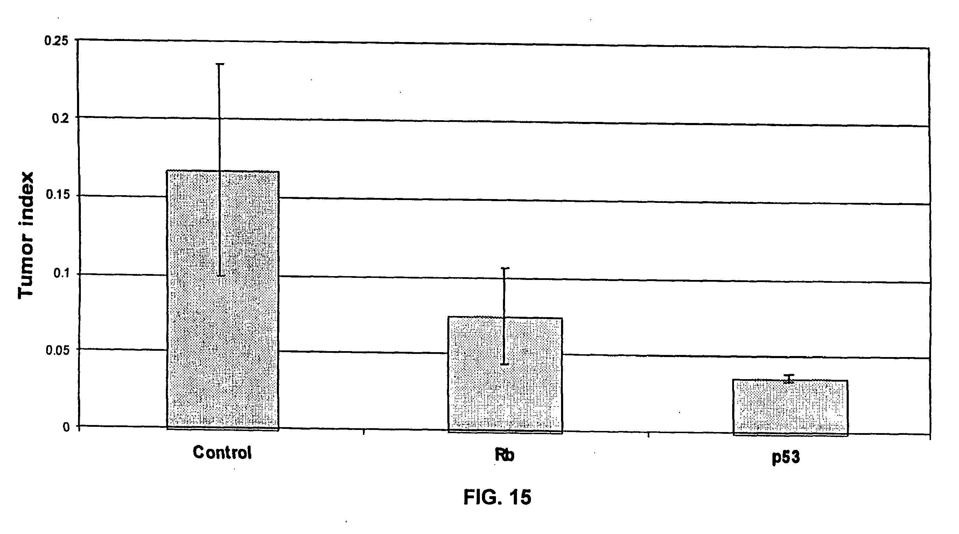

**Figure 15** shows the tumor index for the B 16-F10 melanoma aerosol gene therapy. The tumor cells were injected on day 0 and the treatment started on day 1. PEI-p53 and PEI-RB was delivered twice a week by aerosol for 3 weeks. On day 2 4 post tumor injection, the mice were sacrificed and the lungs weighed. The lung metastasis was graded on a 1-4 points scale. The tumor index was calculated by taking into account the metastasis and the lung weight (p<0.01 vs. control for both the p53 and the Rb group.

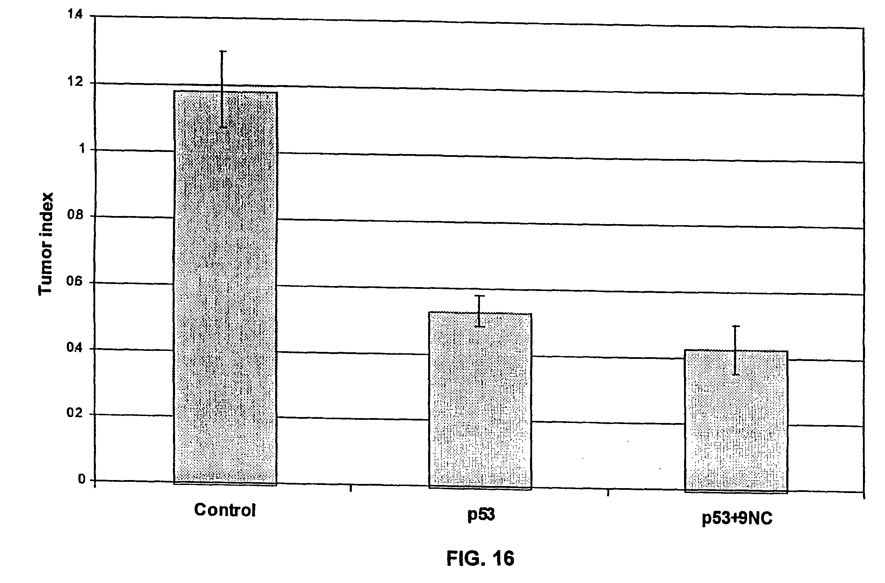

**Figure 16** shows the effects of aerosol combination therapy for M109 adenocarcinoma. The tumor cells were injected on day 0 and the treatment started on day1. The PEI-p53 was delivered twice a week and the drug, 9NC, was also delivered twice a week for 4 weeks. After 4 weeks the mice were sacrificed, the lungs weighed and the tumor foci were counted. Tumor index was calculated taking into account the lung weight, number of tumor foci and the size of tumor foci. ( p<0.0001 vs control for both p53 and p53+9NC group).

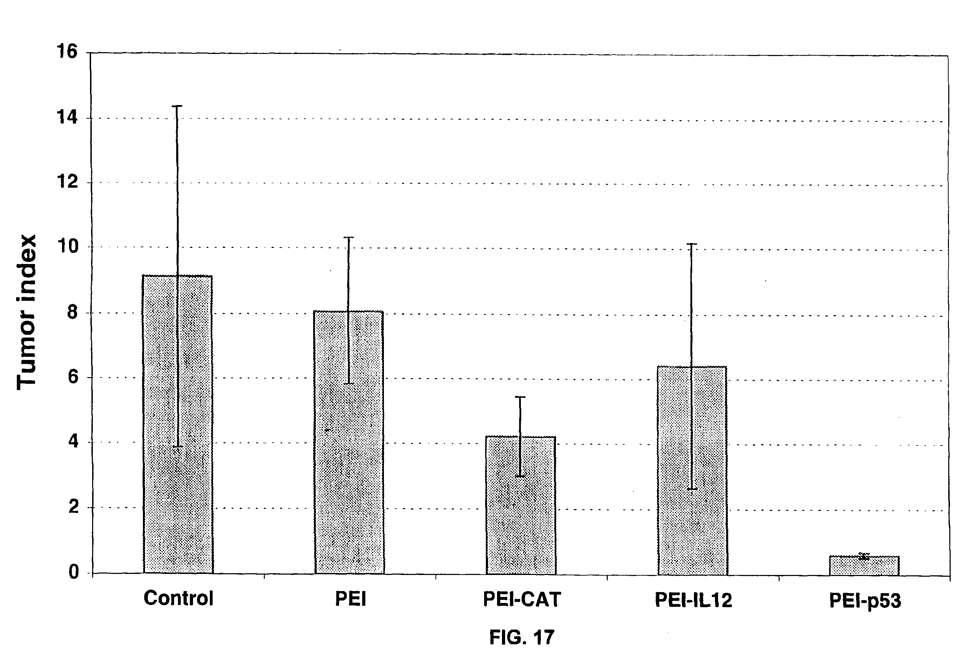

**Figure 17** shows that effects of compositions of PEI and the p53 gene on tumor growth in a nude mouse model of human osteosarcoma lung metastasis.

### DETAILED DESCRIPTION OF THE INVENTION

Aerosol delivery of plasmid DNA to the lungs offers the possibility of direct application of gene preparations to pulmonary surfaces as a means of treating a variety of genetic pulmonary disorders. However, the process of jet nebulization rapidly degrades naked DNA, viral vectors and many lipid-based formulations. While complexing DNA with cationic lipids has been shown to significantly stabilize plasmid DNA, losses of activity occur during nebulization, severely limiting the efficiency of aerosol delivery of many such complexes. In conjunction with the design of aerosol delivery systems appropriate for DNA delivery, formulations using polyethyleneimine (PEI, a polycationic polymer) and macromolecules such as DNA have been developed that result in a high level of pulmonary transfection (10-100-fold greater than cationic lipids) and exhibit a high degree of stability during nebulization procedures. In addition, these polyethyleneimine-based formulations exhibit a high degree of specificity for the lungs compared to a DNA-liposome complex, and are non-toxic. Optimal polyethyleneimine formulations and polyethyleneimine-DNA ratios and concentrations have been determined for both *in vitro* and *in vivo* transfection. The properties of polyethyleneimine-based formulations that make them resistant to nebulization and efficient as DNA delivery vectors for pulmonary sites have been investigated. A heat-labile factor in serum appears to enhance polyethyleneimine-mediated transfection, whereas surfactants have a minimal inhibitory effect. Potential applications of this technology include the use of aerosolized polyethyleneimine-DNA for genetic immunization.

The present invention is intended for delivery via a nebulizer. A number of jet nebulizers are available for single dose or low dose delivery of an aqueous suspension. The polyethyleneimine:DNA formulations of the present invention may also be delivered via ultrasonic nebulization. Additionally, mixtures of polyethyleneimine and DNA may also be dispensed by compressed air or gas in a 'metered dose inhaler'.

Specifically, the present invention is directed towards a method of targeting therapy such as gene therapy via the respiratory tract, comprising the step of: delivering aqueous dispersions of a genetic macromolecule such as a therapeutic gene complexed with polyethyleneimine via small particle aerosol via a respiratory tract of an individual in need of such treatment. Representative examples of genetic macromolecules which may be delivered according to the methods of the present invention include DNA, RNA, catalytically active nucleic acids such as ribozymes, antisense oligonucleotides as well as other types of modified nucleic acids. This method of the present invention may be used to treat an individual having a disease such as cystic fibrosis, asthma, lung cancer, esophogeal cancer, colon cancer, leukemia, breast cancer, sarcoma or melanoma. In addition, the genetic macromolecule complexed with polyethyleneimine in aqueous dispersions may be administered in an air mixture containing up to 10% carbon dioxide gas so as to potentiate the effects of the polyethyleneimine as described in detail below.

The present invention is also directed towards a polycation-genetic macromolecule composition for delivery of a genetic macromolecule such as a therapeutic gene via small particle aerosol, comprising: polyethyleneimine; and a genetic macromolecule. Representative examples of genetic macromolecules which may be contained in such compositions of the present invention include DNA, RNA, catalytically active nucleic acids such as ribozymes, antisense oligonucleotides as well as other types of modified nucleic acids.

The present invention is further directed towards a composition containing a polycation-genetic macromolecule for delivery of a therapeutic gene via small particle aerosol, in which the composition is produced by a method comprising the steps of: dissolving a genetic macromolecule in an appropriate solution; dissolving polyethyleneimine in an appropriate solution; complexing the dissolved genetic macromolecule and the dissolved polyethyleneimine, thereby producing a polyethyleneimine-genetic macromolecule composition; and placing the polyethyleneimine- genetic macromolecule composition in a device capable of nebulization so as to produce a polyethyleneimine-genetic macromolecule composition for delivery of a genetic macromolecule via small particle aerosol. Representative examples of genetic macromolecules which may be contained in such compositions of the present invention include DNA, RNA, catalytically active nucleic acids such as ribozymes, antisense oligonucleotides as well as other types of modified nucleic acids.

Delivery methods for the above-mentioned methods and compositions include oral inhalation and nasal inhalation, by face mask or oral tubes and by oxygen hoods in children, otherwise used to dispense oxygen. Generally, the small particle aerosol is created by a process of nebulization. Preferably, th e process of nebulization is performed by a jet nebulizer. Furthermore, it is desired that the therapeutic gene comprises operably linked elements necessary for expression, and representative therapeutic genes include a cystic fibrosis transmembrane conductance regulator gene, a gene encoding a tumor suppressor, such as p53 or retinoblastoma, or engineered variants of these genes, cytokine genes such as interleukin-2 or interleukin-12, and genes to be used as DNA vaccines that will stimulate a direct immune response against tumors or other infectious agents. Yet another representative therapeutic gene is the HSV thymidine kinase gene. Typically, a final concentration of the therapeutic gene in the polyethyleneimine is no greater than about 10 µg DNA/50 nmol polyethyleneimine nitrogen/ml and no less than about 0.1 µg DNA/50 nmol polyethyleneimine nitrogen/ml. Additionally, serum may be added to the above-described composition. Further, a cell-specific ligand may be covalently conjugated to the polyethyleneimine, and a representative cell-specific ligand is transferrin.

In accordance with the present invention, there may be employed conventional molecular biology, microbiology, and recombinant DNA techniques within the skill of the art. Such techniques are explained fully in the literature. See, e.g., Sambrook, Fritsch & Maniatis, "Molecular Cloning: A Laboratory Manual (1982); "DNA Cloning: A Practical Approach," Volumes I and II (D.N. Glover ed. 1985); "Oligonucleotide Synthesis" (M.J. Gait ed. 1984); "Nucleic Acid Hybridization" [B.D. Hames & S.J. Higgins eds. (1985)]; "Transcription and Translation" [B.D. Hames & S.J. Higgins eds. (1984)]; "Animal Cell Culture" [R.I. Freshney, ed. (1986)]; "Immobilized Cells And Enzymes" [IRL Press, (1986)]; B. Perbal, "A Practical Guide To Molecular Cloning" (1984). Therefore, if appearing herein, the following terms shall have the definitions set out below.

A "DNA molecule" refers to the polymeric form of deoxyribonucleotides (adenine, guanine, thymine, or cytosine) in its either single stranded form, or a double-stranded helix. This term refers only to the primary and secondary structure of the molecule, and does not limit it to any particular tertiary forms. Thus, this term includes double-stranded DNA found, *inter alia,* in linear DNA molecules (e.g., restriction fragments), viruses, plasmids, and chromosomes.

A "genetic macromolecule" shall include DNA, including therapeutic genes, RNA, catalytically active nucleic acids such as ribozymes, antisense oligonucleotides as well as other types of modified nucleic acids.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site, as well as protein binding domains (consensus sequences) responsible for the binding of RNA polymerase. Eukaryotic promoters often, but not always, contain "TATA" boxes and "CAT" boxes. Prokaryotic promoters contain Shine-Dalgarno sequences in addition to the -10 and -35 consensus sequences.

A DNA "coding sequence" is a double-stranded DNA sequence which is transcribed and translated into a polypeptide *in vivo* when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (e.g., mammalian) DNA, and even synthetic DNA sequences.

Transcriptional and translational control sequences are DNA regulatory sequences, such as promoters, enhancers, polyadenylation signals, terminators, and the like, that provide for the expression of a coding sequence in a host cell. A "cis-element" is a nucleotide sequence, also termed a "consensus sequence" or "motif", that interacts with other proteins which can upregulate or downregulate expression of a specific gene locus. A "signal sequence" can also be included with the coding sequence. This sequence encodes a signal peptide, N-terminal to the polypeptide, that communicates to the host cell and directs the polypeptide to the appropriate cellular location. Signal sequences can be found associated with a variety of proteins native to prokaryotes and eukaryotes. A polyadenylation signal and transcription termination sequence will usually be located 3 ' to the coding sequence.

An "expression control sequence" is a DNA sequence that controls and regulates the transcription and translation of another DNA sequence. A coding sequence is "operably linked" and "under the control" of transcriptional and translational control sequences in a cell when RNApolymerase transcribes the coding sequence into mRNA, which is then translated into the protein encoded by the coding sequence.

A "gene" may comprise an intact gene as the gene occurs in nature, i.e. a coding sequence driven by promoter and/or additional sequences as found in nature. Alternately, a gene may comprise a coding sequence and a heterologous promoter (with or without heterologous transcriptional and/or translational control sequences) to drive expression of the coding sequence. As used herein, a "therapeutic gene" is typically a gene, either with it's native promoter or a heterologous one driving expression, encoding a protein that has a therapeutic effect, *e.g.* a gene encoding a required enzyme otherwise absent from a cell.

In general, expression vectors containing promoter sequences which facilitate the efficient transcription and translation of the inserted DNA fragment are used in connection with a host. The expression vector typically contains an origin of replication, promoter(s), terminator(s), as well as specific genes which are capable of providing phenotypic selection in transformed cells. The transformed hosts can be fermented and cultured according to means known in the art to achieve optimal cell growth.

A "heterologous' region of the DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. In another example, the coding sequence is a construct where the coding sequence itself is not found in nature (e.g., a cDNA where the genomic coding sequence contains introns, or synthetic sequences having codons different than the native gene). Allelic variations or naturally-occurring mutational events do not give rise to a heterologous region of DNA as defined herein.

A "vector" is a replicon, such as plasmid, phage or cosmid, to which another DNA segment may be attached so as to bring about the replication of the attached segment. A "replicon" is any genetic element (e.g., plasmid, chromosome, virus) that functions as an autonomous unit of DNA replication *in vivo;* i.e., capable of replication under its own control. An "origin of replication" refers to those DNA sequences that participate in DNA synthesis.

As used herein, the term "aerosol" refers to dispersions in air of solid or liquid particles of fine enough size and consequent low settling velocities to have relative airborne stability (Knight, V., Viral and Mycoplasmal Infections of the Respiratory Tract, 1973, Lea and Febiger, Phila. PA, pp.2).

As used herein, the term "nebulization" or "process of nebulization" refers to the production of fine aerosols from a liquid supply, i.e., the product of a refluxing, baffled spray cloud-producing device (May, 1973).

As used herein, the term "jet nebulizer" refers to a device in which compressed air expands into a nozzle with resulting reduction of static pressure which sucks fluid from the nebulizer reservoir through an attached tube. This fluid is broken up by the air jet into a dispersion of droplets of a wide size range. Most of the droplets (about 99.92%) are impacted on the wall of the enclosing flask and reflux to the reservoir. The remaining small percentage (about 0.02%) escapes impact and flows out of the nebulizer carried by the compressed air stream. These constitute the fine droplet aerosol.

As used herein, "intranasal instillation" is a process whereby liquid is applied to the nose of an anesthetized animal, which is an obligate nasal breather, so that the animal subsequently inhales the liquid into the airways or lungs.

It is specifically contemplated that pharmaceutical polyethyleneimine (PEI):DNA compositions of the present invention may be prepared for aerosol delivery of aqueous dispersions to the respiratory tract of an individual or animal. A person having ordinary skill in this art would readily be able to determine, without undue experimentation, the appropriate dosages of the aerosol formulations. When used *in vivo* for targeted gene therapy, the polyethyleneimine:DNA composition of the present invention is administered to the patient or an animal in therapeutically effective amounts, i.e., amounts that efficiently express the therapeutic gene and thereby eliminate or reduce the disease. The dose and dosage regimen will depend upon the nature of the disease, the characteristics of the particular polyethyleneimine:DNA composition, *e.g.*, its therapeutic index, the patient, the patient's history and other factors. The amount of polyethyleneimine:DNA composition administered will typically be in the range of about 0.01 to about 1.0 mg/kg of patient weight. The schedule will be continued to optimize effectiveness while balanced against negative effects of treatment. *See* Remington's Pharmaceutical Science, 17th Ed. (1990) Mark Publishing Co., Easton, Penn.; and *Goodman and Gilman's: The Pharmacological Basis of Therapeutics* 8th Ed (1990) Pergamon Press; which are incorporated herein by reference. The composition or formulation may contain minor amounts of additives, such as substances that enhance isotonicity and chemical stability, *e.g.,* buffers and preservatives. The polyethyleneimine:DNA composition will typically be formulated at concentrations of about 0.1 µg DNA/20 nmoles PEI nitrogen/ml to 10 µg DNA/150 nmoles PEI nitrogen/ml.

The following examples are given for the purpose of illustrating various embodiments of the invention and are not meant to limit the present invention in any fashion:

### EXAMPLE 1

### PEI-DNA Formulations

PEI (25kD) was obtained from Aldrich Chemical (Milwaukee, WI). A stock solution of PEI was prepared at a concentration of 4.3 mg/ml (0.1 M in nitrogen) in phosphate buffered saline (PBS). DNA contains 3 nmols of phosphate per µg, and appropriate concentrations were prepared in PBS. The desired amount of DNA in PBS was complexed with PEI by slowly adding the DNA to the PEI while vigorously vortexing the solution. The solution was then allowed to incubate at room temperature for 15-20 min prior to use. The resulting charge ratio is expressed as PEI nitrogen:DNA phosphorous (N:P).

### EXAMPLE 2

### Plasmid DNA

The cytomegalovirus promoter (CMV) with the *E. coli* β-galactosidase reporter gene (pCMVβ; CLONTECH, Palo Alto, CA) and jellyfish green flourescent protein gene (pEGFP; CLONTECH) were used to assess mammalian cell expression *in vitro. In vivo* transfection was assessed with the chloramphenicol acetyltransferase gene (pCMVHiCAT) (a gift from Genzyme, Inc., Framingham, MA). The luciferase plasmid (pGL3, Promega, Madison, WI) modified by insertion of the CMV promoter/enhancer and the human growth hormone polyadenylation sequence (from Michael A. Barry at Baylor) was used as a reporter gene for both *in vitro* and *in vivo* transfection. The plasmid used for immunization studies, pCMV-hGH, expresses human growth hormone and was also obtained from Michael A. Barry. Bacterial cultures were transformed with the above plasmids and bulk quantities of plasmid DNA were produced and purified using reagents and columns for endotoxin free DNA (Qiagen, Valencia, CA), and then dissolved in endotoxin free water to the desired concentration.

### EXAMPLE 3

### Synthesis of Cationic Lipids

Cationic lipids were either synthesized or were gifts. DC-cholesterol (3(-[N-[(N',N'-dimethyl-amino) ethane] carbamoyl] cholesterol) was synthesized according to the methods of Gao and Huang (1991). Guanidinium cholesterol (bis-guanidinium-tren-cholesterol; BGTC) was synthesized according to the methods of Vigneron et al. (1996). N-(2-hydroxyethyl)-N,N-dimethyl-2,3-bis (tetradecytoxy)-1-propanaminium bromide (DMRIE) was obtained from Vical (San Diego, CA).

### EXAMPLE 4

### Preparation of Cationic Lipid:Neutral Co-lipid-DNA complexes

All of the cationic lipid:DOPE formulations were first prepared by mixing the cationic lipid (5 mg/ml in chloroform) with an appropriate volume of dioleoyl phosphatidylethanolamine (DOPE; Avanti Polar Lipids; 5 mg/ml in chloroform) or cholesterol (5 mg/ml in chloroform; Sigma) and drying under nitrogen. The lipid mixture was dissolved in t-butanol at 37°C, frozen at -80°C, and lyophilized for at least 24 h. The lipids were stored at -20°C until use, at which time they were warmed to room temperature and resuspended in sterile water (Water for Irrigation (WFI; Baxter, Deerfield, IL)) for at least 30 min prior to complexing with DNA. Lyophilized BGTC:DOPE formulations appeared to be stable at -20°C over a period of 6-8 months or more.

### EXAMPLE 5

### Tissue Culture

The A549 human lung carcinoma cell line was purchased from the American Type Culture Collection (Rockville, MD). Cells were cultured in Dulbecco's Modified Essential Medium (D-MEM) supplemented with 10% defined fetal bovine serum (FBS), 2 mM L-glutamine, and 50 µg/ml gentamicin (unless otherwise noted) or in RPMI 1640 (Gibco) with 10% FBS. Cells were cultured in a cell culture incubator at 37°C and in the presence of 5% CO₂. Only the first 8 passages of A549 cells were used.

### EXAMPLE 6

### In vitro Transfection

For serum-free transfections, cells were plated at 1.5 X 10⁵ cells/well in 12 well plates the day prior to transfection (unless otherwise noted). Cells were approximately 80% confluent at the time of transfection. After PEI-DNA or lipid-DNA complexing was complete, the PEI-DNA or liposomal-DNA formulations were brought up in Opti-mem I Reduced Serum Medium (Opti-mem; Gibco-BRL, Grand Island, NY) to a final DNA concentration of 1 µg/ml. Immediately prior to transfection, cells were rinsed with Opti-mem and then overlaid with 1 ml of the transfection solution per well (3 wells were generally transfected for each time point or condition). Plasmid-PEI complexes were prepared at various nitrogen to phosphate (N:P) ratios based on the composition of PEI (25000 MW; 581 moles of nitrogen per mole) and DNA (3 nmol of phosphate per µg). After 24 h (except where a shorter transfection time is indicated), the transfection solution was removed, the cells were rinsed, and 1 ml of supplemented DMEM medium was added. The cells were then incubated for an additional 24 h, rinsed two times with phosphate buffered saline (PBS) and lysed using a lysis buffer (0.1 M Tris-HCl, 0.5% Triton X-100 (Sigma, St. Louis, MO), and assayed for β-galactosidase or luciferase expression.

In order to determine optimal polyethyleneimine nitrogen to DNA phosphate (N:P) ratios and the optimal concentration of polyethyleneimine nitrogen for *in vitro* applications in the presence of serum, the following protocol was developed: 1) PEI-DNA complexes were prepared in a range of N:P ratios and allowed to incubate at room temperature for 15-20 min prior to use; 2) Cells were plated at 10⁵ cells/well in a 24 well format and incubated at 37°C overnight in 1 ml of RPMI 1640 medium supplemented with 10% FBS; 3) The volume of medium in each well was then adjusted to give the desired final concentration of PEI nitrogen; 4) A constant volume of PEI-DNA solution was added to each well for each particular N:P ratio; 5) The plates were incubated at 37°C overnight, and inspected for GFP expression or assayed for luciferase expression.

### EXAMPLE 7

### Effect of serum, surfactant and bronchoalveolar lavage fluid on in vitro PEI transfection

A549 cells were plated and transfected as described above except that instead of diluting the complexed formulations with Opti-mem prior to transfection, they were diluted with Optimem containing the reagent of interest (serum, surfactant, or bronchoalveolar lavage fluid). Fetal bovine serum (FBS) was obtained from HyClone Laboratories, Inc. (Logan, UT), mouse and human serum were obtained from Gibco-BRL, and synthetic surfactant (Exosurf, containing primarily colfasceril palmitate at a concentration of 81 mg/ml) was obtained from Burroughs Welcome Co. (Research Triangle, N.C.). Bronchoalveolar lavage (BAL) fluid was obtained from untreated mice that were sacrificed by methoxyflurane anesthesia and exsanguinated via the abdominal aorta. The trachea was surgically exposed and canulated with PE50 tubing (outer diameter 0.965 mm, Clay Adams). Using a total volume of 2.0 ml physiological saline, the lungs were lavaged 5 times with approximately 1.0 ml volume (a 1.0 ml volume of the same fluid was pumped into and pulled out t of the lungs 5 times using an attached 3 ml syringe holding a total of 2 ml). The yield is typically 85% recovery of the 2 ml of lavage fluid. The BAL fluid pooled from several mice was then lyophilized and reconstituted at different concentrations for the transfection analysis.

### EXAMPLE 8

### Quantitative Analysis of in vitro Transfection Efficiency

After lysis of the cells, the protein concentration of the lysate was determined using the BCA Protein Assay from Pierce (Rockford, IL). The β-galactosidase activity was quantified by using a chlorophenolred-β-D-galactopyranoside (CPRG; Boehringer Mannheim, Indianapolis, IN) assay in a 96 well plate format and read on a Dynatech MR5000 microtiter plate reader (Dynatech Laboratories, Inc., Chantilly, VA).

Luciferase assays were performed with commercial reagents according to the manufacturers directions (Promega, Madison, WI). For luciferase expression in cell cultures, the medium was removed and 1 ml of lysis buffer (Promega, Madison, WI) was added to each well. After incubation of the plate for 2 0 min at room temperature on a rotary agitator, the lysates were assayed by mixing 1-10 µl of lysate with 50 µl substrate and measuring light output for 15 sec in a TD-20C Luminometer (Turner Designs, Sunnyvale, CA).

### EXAMPLE 9

### Functional Stability of Nebulized PEI-DNA and Liposome-DNA Complexes

The PEI-DNA and lipid-DNA formulations were prepared by diluting the DNA to 80 µg/ml in 2.5 ml WFI, adding the appropriate amount of lipid in 2.5 ml WFI to the DNA or the appropriate amount of DNA to PEI in PBS and incubating at room temperature for 15-30 min before nebulizing. At the time intervals indicated, 50-75 µl aliquots were removed from the nebulizer reservoir and prepared for *in vitro* transfection. pCMV-β was used as the reporter gene in all of these studies and the functional stability of the plasmid DNA was analyzed in A549 cells in 12 well plates as described above. It should be noted that for jet nebulizers to produce small particle aerosols they must recycle nebulizer liquid continuously, since only a minute fraction of the nebulizer fluid (less than 1 %) is released as aerosol with each pass through the nebulizer jet. Large lipid-DNA particles (>400 nm) are rapidly processed into smaller sizes so that within a short time, the lipid-DNA particle size within the nebulizer reservoir becomes very similar to that of aerosol particles recovered from aerosol in the All Glass Impinger (AGI-4, Ace Glass Co., Vineland, NJ)).

The Puritan Bennett 1600 twin jet nebulizer (Carlsbad, CA) used in this study was modified by removing one tube from one of the twin jets and is referred to as the Puritan Bennett 1600 single jet (PB sj). The PB sj was run at a flow rate of 15 L/min unless otherwise indicated.

### EXAMPLE 10

### In Vivo Transfection with PEI-DNA and Liposome-DNA by Instillation

PEI-DNA and lipid-DNA formulations were prepared as described herein except that the concentration of DNA was increased to 6 µg per 40 µl (after addition of the appropriate concentration of liposomes or adding to the appropriate concentration of polyethyleneimine) for one instillation dose. In order to minimize variability resulting from an occasional incomplete instillation (animals sometimes discharge part of the instilled material), each animal was instilled a total of four times: once in the morning and once in the afternoon on each of two consecutive days using the same amount of DNA. Animals were lightly anesthetized with methoxyflurane immediately prior to administration of the formulation. While animals were held vertically upright by the scruff of the neck, 40 µl was applied slowly to one nostril where the fluid was inhaled. The animals were maintained in an upright position for a short period of time to allow instilled fluid to reach the lungs. The efficiency of lung delivery using this approach has been verified using radiolabeled liposomal preparations and most of the material is found in the lungs after a few minutes. Animals exhibit no obvious signs of physiological impairment or distress as a result of this treatment and no obvious pathological consequences were noted upon gross examination of lungs and other organs. Since previous work has shown the optimal time for expression of instilled reporter genes to be 1-2 days postinstillation, animals were sacrificed 48 h after initiation of the first instillation.

### EXAMPLE 11

### In Vivo Transfection with PEI-DNA and Liposome-DNA by Aerosol

PEI-DNA and lipid-DNA formulations were prepared as described herein except that the concentration of DNA was increased to 2 mg per 20 ml (after addition of the appropriate concentration of liposomes or polyethyleneimine) for one nebulizer dose. Unless otherwise stated, the PB sj nebulizer was run at a flow rate of 8 L/min. The outflow of the nebulizer was passed through a sealed plastic cage (13x17x30 cm.) which housed the mice (usually 6-8). Air exited this chamber through a HEPA filter and the entire apparatus was situated under a laminar flow hood vented through additional HEPA filtration to the outside. To minimize the use of aerosol, the nebulizer was operated under automatic control for only 1 min in each 10 min. When the first 20 ml of suspension was consumed, the nebulizer was replenished with an additional 20 ml at the same concentration. The exposure required approximately 12 h, during which time the mice were supplied with food and water and allowed to move freely about the chamber.

### EXAMPLE 12

### Quantitative Analysis of Gene Expression in vivo

CAT expression was found to be a more sensitive means of measuring transfection efficiency *in vivo* than β-galactosidase, which was used for *in vitro* studies. Animals that were exposed to nebulized formulations were sacrificed at appropriate time points (48 h unless otherwise indicated) and the lungs and trachea were removed and frozen in liquid nitrogen. The tissues were later minced in lysis buffer and homogenized using a Wig-L-Bug bead homogenizer (Crescent Dental Mfg., Lyons, IL). For the determination of transfection in the nose, the nose section of the mouse skull was cut away from the rest of the skull and frozen in liquid nitrogen. The frozen nose section was pulverized and then subjected to homogenization in lysis buffer using the Wig-L-Bug bead homogenizer. The tissue extracts were analyzed for CAT content using a commercially available CAT ELISA kit (Boehringer Mannheim Gmbh, Germany). The results obtained with the ELISA assay were equivalent or better than those obtained using conventional TLC methods and organic phase separation methods. Because of the variability observed with virtually all *in vivo* aerosol studies, experimental and control groups usually consisted of 6-8 animals.

For *in vivo* analysis of luciferase expression after instillation or aerosol delivery, animals were sacrificed by lethal anesthesia, exanguinated by transection of the abdominal aorta and the lungs and the trachea harvested. In some studies, the nasal tissues were also harvested. The tissue was homogenized in 1 ml of luciferase assay lysis buffer using a conical ground glass tissue grinder (Kontes Duall 23). A 10 µl aliquot of the homogenate was added to 50 µl of substrate and light output was measured.

### EXAMPLE 13

### Genetic immunization

Groups of 5 mice were exposed to pCMV-hGH combined with PEI (N:P ratio of 10) via aerosol or to intranasal plasmid. As a positive control group, five mice received naked plasmid (50 µg in 50 µl PBS) as an intramuscular injection in the anterior tibialis muscle. Sequential serum samples were obtained at bi-weekly intervals, and assayed by ELISA for total specific antibody to human growth hormone (purified hGH protein was obtained from Cal-Biochem, San Diego, CA). A pool of sera from unimmunized mice served as a negative control in each ELISA.

### EXAMPLE 14

### In vitro Optimization of PFT-DNA Charge Ratios and Concentrations

Because of the importance of charge ratio to the transfection efficiency of most polycation-DNA formulations, the relationship between the PEI-DNA ratio and transfection was examined *in vitro* using A549 cells in the absence of serum. As shown in Figure 1A, a polyethyleneimine nitrogen to DNA phosphate (N:P) ratio of about 5 is optimal for *in vitro* transfection with the pCMVβ plasmid, as determined by expression of β-galactosidase. Transfection drops off rapidly at ratios below 4 and declines as the ratio increases above 5. Very similar results were obtained with PEI-luciferase formulations (Figure 1B). When the effects of these ratios on protein levels of cell extracts from the same experiments (which correlates roughly with cell density) were examined, little or no toxicity was evident at ratios below 8 (Figure 1C). Decreasing protein levels for groups treated with higher ratios is probably indicative of cellular toxicity.

Since *in vivo* transfection must occur in the presence of extracellular fluid proteins, and because of the toxicities noted above, the effects on transfection efficiency of both the concentration of polyethyleneimine and the N:P ratio were examined using A549 cells in culture medium containing 10% fetal calf serum. Figure 2 shows that an N:P ratio of 16 with a PFI concentration of 50-60 nmol/ml gives the highest degree of transfection, indicating that both the concentration of polyethyleneimine and the ratio of polyethyleneimine to DNA strongly influence transfection efficiency in the presence of serum proteins. Of particular interest is that the optimal N:P ratio is higher in the presence of serum than in its absence (Figure 1B). The overall transfection efficiency with optimal conditions in the presence of serum was actually higher than in its absence. The same optimization protocol was used in other cells, indicating that this relatively simple technique is useful for customizing PEI transfection conditions for a particular application.

### EXAMPLE 15

### Effects of Serum and Surfactants on PEI-DNA Transfection Efficiency In vitro

Although previous reports have shown that serum proteins tend to inhibit transfection mediated by a variety of cationic lipids and polycations, the enhancement observed herein under optimal polyethyleneimine concentration and N:P ratio conditions led to the investigation of the effects of serum on polyethyleneimine's transfection efficiency. When fetal bovine serum concentrations of 0 to 50% were examined, it was evident that concentrations of 5% to 20% enhanced transfection *in vitro,* while higher concentrations (50%) resulted in reduced transfection (Figure 3). The inhibition associated with high concentrations of serum could be partially overcome with increasing concentrations of PEI:DNA. There was some species specificity of the serum effect, with fetal bovine and human serum both resulting in a similar enhancement of transfection, while mouse serum had no effect. Rabbit serum also lead to an enhancement of PEI-mediated transfection. The serum effect appeared to be heat labile with a complete loss of enhancing activity occurring on heating to 95° for 10 min, although heating for 30 min at 56° in subsequent experiments did not eliminate the enhancing effect.

The effect of pulmonary surfactant on PEI-mediated transfection was also investigated. This was performed with bronchoalveolar lavage fluid from mice (lyophilized and rehydrated at increasing concentration) and synthetic surfactant (Exosurf) at increasing concentrations. Cells were exposed to concentrations of BAL fluid equivalent to 6.25% to 125% of that of the unlyophilized BAL fluid (the concentrated BAL fluid added to each well equaled 0.5 to 10% of the total volume of the fluid in each well respectively). Cells were exposed to concentrations of Exosurf equivalent to 4.05 up to 81 µg/ml of colfasceril palmitate, the active agent in Exosurf. As shown in Figure 4, higher concentrations of both BAL fluid and Exosurf resulted in decreased levels of transfection in contrast to the effects of serum, while lower concentrations of surfactant appeared to have little effect.

### EXAMPLE 16

### Effect of Nebulization on in vitro Transfection Efficiency of liposome-DNA and PEI-DNA Complexes

Preparations of PEI-DNA complexes at N:P ratios of 5 and 10 were tested for transfection efficiency over a 10 min period of nebulization. In one experiment, the use of a PEI-DNA formulation (N:P=10) resulted in an average of 96%, 94% and 89.6% retention of the transfection activity of the control (unnebulized) material in an *in vitro* assay using A549 human lung tumor cells. Aliquots of the nebulizer-cycled material were removed from the nebulizer reservoir (with a starting volume of 5 ml) at 3 min intervals. For comparison, the preparations of PEI-DNA, a preparation of DC-cholesterol:DOPE:pCMVβ (2:2:1) and a preparation of DMRIE:DOPE:pCMVβ (3:3:1) were nebulized to dryness (approximately 12 min) (Figure 5). The formulations of DC-cholesterol:DOPE and DMRIE:DOPE used herein were optimized for *in vitro* transfection of A549 cells and the ratio of DC-cholesterol:DOPE used is similar to that reported elsewhere for optimal cell culture transfection (Gao & Huang, 1991). There was only a slight loss of the β-galactosidase activity of the PEI-pCMVβ (N:P=10) preparation at a nebulizer flow rate of 8 L/min, whereas the DC-cholesterol:DOPE:pCMVβ preparation lost 60% of its activity in 3 min and was virtually inactive thereafter. The activity of DMRIE:DOPE:pCMVβ was reduced by over 70% after 3 min and about 90% after 6 min. At higher nebulizer flow rates, the loss of transfection efficiency with formulations involving other cationic lipids (Schwartz et al., 1996) is even greater. Interestingly, the N:P=5 preparation of PEI-pCMVβ exibited a pronounced increase in transfection efficiency with increased nebulization, which might reflect degradation of part of the DNA, thereby effectively increasing the N:P ratio.

### EXAMPLE 17

### Comparison of in vivo Transfection Efficiency of Liposome-DNA and PEI-DNA Complexes After Intranasal Instillation

When PEI:pCMV-HiCAT formulations were tested *in vivo* by intranasal instillation of Balb/c mice (Figure 6A), it was discovered that CAT expression resulting from PEI-DNA was 10-20 times greater in lung than that obtained using BGTC:DOPE:pCMV-HiCAT, the most stable and effective cationic lipid formulation for *in vivo* transfection to date. PEI-mediated expression appeared to be more specific for the lungs, where transfection was about 20-fold higher than in the nose. In contrast, expression mediated by instilled cationic lipid-based formulations was higher in the nose than in the lungs. Similar data was obtained when animals were instilled with PEI-luciferase or r BGTC:DOPE-luciferase (Figure 6B).

Intranasal instillation was also used to compare the transfection efficiencies *in vivo* of different formulations of PEI-pCMV-HiCAT. When N:P ratios of 5 to 20 were examined, it was found that ratios of 10 and 20 resulted in comparable levels of *in vivo* expression of CAT. However, when the concentration of PEI-DNA (at an N:P ratio of 10) was increased several-fold, thereby allowing the delivery of a 3-fold higher dose of DNA in the same instillation volume, no further increase in *in vivo* reporter gene expression was observed.

### EXAMPLE 18

### Comparison of in vivo Transfection Efficiency of Liposome-DNA and PEI-DNA Complexes After Aernsol Delivery

When PEI-pCMV-HiCAT and BGTC:DOPE-pCMV-HiCAT formulations (using identical concentrations of DNA) were delivered by nebulization, polyethyleneimine was a 10-fold better vector for expression in lung than the lipid (Figure 7), confirming the above findings obtained by instillation. Also in agreement with the instillation findings was the observation that aerosol delivery resulted in about 20-fold greater transfection in the lungs than in the nose for PEI-based formulations, while aerosolized BGTC:DOPE-pCMVβ resulted in comparable levels of expression in the nose and lungs. The aerosol delivery of PEI-based formulations of luciferase again resulted in similar findings regarding their relative potencies in lung and nose.

### EXAMPLE 19

### Genetic Immunization by Aerosol and Instillation Administration of PEI-DNA Vaccines

One potentially valuable application for aerosol administration of expression vectors is the delivery of DNA vaccines. To determine whether immunization could be achieved by this method, the pCMV-hGH plasmid-PEI preparation (N:P ratio of 10) was administered via aerosol and nasal instillation, and, as a positive control, intramuscular injected with naked DNA. As shown in Figure 8, the treatments elicited high levels of serum antibody to human growth hormone in all groups. A substantial antibody response was present at 2 weeks and plateaued between 4 and 8 weeks. In Figure 8, the optical density is shown at a 1:500 dilution of serum, as compared to a background OD of 0.1 in unimmunized animals. The mean titers of these sera at 8 weeks were 1:4,000 for the aerosol group, 1:8,000 for the intranasal and 1:16,000 for the intramuscular groups, respectively. As shown in Figure 8, however, all groups had fairly large standard deviations, similar to prior results with intramuscular injections. Thus, although the responses of individual mice were highly variable, the persistence of antibody through 8 weeks from a single administration of the plasmid-PEI preparation suggests that this method of immunization has significant application when given by the intranasal or the aerosol route.

The efficient delivery of genetic material to the lungs and airways represents a targeted and noninvasive approach to the treatment of a wide range of pulmonary disorders. However, transfection of pulmonary sites has generally been inefficient when using nonviral DNA delivery vectors to minimize the pathologic and immune concerns associated with viral delivery. This has been due, in part, to the inaccessibility of the pulmonary environment to delivery of gene preparations. In addition, the loss of activity associated with jet nebulization has limited the value of this methodology for delivery of DNA to pulmonary targets (Crook et al., 1996; Schwartz et al., 1996). The current study found that complexes of DNA with the polycationic polymer polyethyleneimine have substantial functional stability when delivered by jet nebulization, thus providing a potential means of gene delivery to the lung.

Other nonlipid polycationic polymers that have been used as DNA delivery vectors include poly-L-lysine, which mediates only a low degree of transfection, but is significantly improved by conjugation or incorporation of agents to facilitate cellular uptake (Wu & Wu, 1988; Tang & Szoka, 1997), and polyamidoamine dendrimers (Haensler & Szoka, 1993). In addition, modified forms of PEI, covalently conjugated with ligands such as transferrin (Kircheis et al., 1997; Ogris et al., 1998; Ogris et al., 1999), have been shown to target certain cell types with a high degree of specificity. There have even been reports of PEI-mediated transfection in lung when formulations were administered systemically (Goula et al., 1998), but to date, little attention has focused on the use of nonlipid polymers as DNA vectors for aerosol delivery. The findings presented herein show that PEI-based formulations lend a remarkable degree of stability to plasmid DNA during the process of jet nebulization. In the case of some PEI-based formulations, transfection even appears to be enhanced by the process of nebulization. It thus appears to be a more effective mediator of transfection after delivery to the lung by aerosol than cationic lipids and has the additional advantage that it is relatively nontoxic. The fact that polyethyleneimine can be readily conjugated to site-specific ligands should increase the versatility of aerosol delivered PEI-based formulations

The enhancement of PEI-mediated transfection by heat labile serum factor(s) is intriguing, although the mechanism(s) involved or the consequences for *in vivo* gene delivery are currently unknown. The fact that the effect is lost when the serum is heated to 100°C argues against the possibility that it is related to small, heat stabile factors such as steroids. The fact that the enhancement is unaffected at 56°C argues against a role for the complement system. The identification of this transfection-enhancing factor will eventually prove useful in improving the overall efficiency of *in vivo* gene delivery.

The different regional specificities (lung vs. nose) of polyethyleneimine and cationic lipid (BGTC:DOPE) noted when using two separate reporter genes is an unexpected finding. Assuming that the particle sizes delivered from the same nebulizer are similar, the differences observed may be due to differences in the response of the two kinds of particles to mucociliary action or due to differences in cellular response at the nasal and pulmonary sites. This finding could be important in applications where nasal expression of the delivered gene may have unwanted consequences. On the other hand, these different delivery vectors may be suitable for different clinical applications. In the area of genetic immunization, the findings reported herein suggest that persistent, high level antibody responses can be achieved with a single administration of a plasmid using techniques that deliver the PEI-DNA complex to pulmonary tissue.

Since PEI-DNA formulations can be efficiently deposited in the lung periphery by aerosol (Weibel generations 17-23), PEI-mediated transfection of the respiratory epithelium in *vivo* will require exposure of plasmid DNA and PEI to endogenous surfactant lipids and proteins in the alveolar space. The effects of surfactants on PEI-mediated transfection were examined in *vitro* using both synthetic surfactant (Exosurf) and bronchoalveolar lavage fluid derived from untreated mice. While both of these substances did result in a concentration-dependent inhibition of PEI-mediated transfection, the effect of surfactants may be relatively lower than those reported for cationic liposome-mediated gene transfer (Duncan et al., 1997; Tsan et al, 1997). These reports teach that tracheal insufflation of plasmid DNA and plasmid DNA-cationic liposome complexes resulted in similar amounts of gene expression in the lung. A much higher level of lung expression is achieved with PEI-pCMV-HiCAT than with plasmid-DNA alone by instillation, providing evidence that pulmonary surfactants do not neutralize the gene transfer properties of polyethyleneimine to the extent that they apparently do for some cationic lipids or cationic lipid:co-lipid formulations. Thus, PEI-based formulations are a promising alternative to the relatively inefficient cationic lipids as nonviral carriers for the treatment of a variety of pulmonary disorders by aerosol. The results presented herein also show promise for the delivery of genetic vaccines.

### EXAMPLE 20

### Animals

Female Balb/C mice (5-7 weeks old) were used in following experiments.

### EXAMPLE 21

### Plasmid DNA

The bacterial chloramphenicol acetyl transferase gene (p4119, ref.15) was primarily used as the reporter gene for measuring transgene expression. The CAT gene is under the control of the human cytomegalovirus (CMV) early promoter/enhancer element. The luciferase plasmid (pGL3, Promega, Madison, WI) modified by insertion of the CMV promoter/enhancer element and the human growth hormone polyadenylation sequence was from Dr. Michael Barry (Center for Cell and Gene Therapy, Baylor). All plasmids were purified on Qiagen columns (Qiagen, Valencia, CA) and were endotoxin free. The plasmids were quantitated by UV absorbance at 260 nm. Agarose gel analysis revealed the plasmids to be a mixture of primarily supercoiled plasmid with a small amount of nicked plasmid.

### EXAMPLE 22

### Preparation of PEI-DNA complexes

PEI (25 KDa, branched) was purchased from Aldrich Chemical (Milwaukee, WI). A polyethyleneimine stock solution was prepared at a concentration of 4.3 mg/ml (0.1 M in nitrogen) in PBS, pH 7-7.5. PEI and DNA were mixed separately in 5 ml water at the required concentrations. The PEI solution was slowly vortexed and the DNA solution was added to it to make a final volume of 10 ml. The mixture was allowed to stand at room temperature for about 15-20 minutes before nebulization. The resulting charge ratio is expressed as PEI nitrogen:DNA phosphate (N:P), which can be calculated by taking into account that DNA has 3 nmol of phosphate per µg and 1 µl of 0.1 M PEI solution has 100 nmol of amine nitrogen. A 10:1 N:P ratio corresponds to 1.29:1 PEI:DNA weight ratio.

### EXAMPLE 23

### Aerosol delivery of PEI-DNA complexes

Mice were placed in plastic cages that were sealed with tape before aerosol delivery (16). This is an unrestrained, whole body aerosol exposure system. PEI-DNA complexes were aerosolized using an Aerotech II nebulizer (AT-II) (CIS-US, Inc., Bedford, MA) at 10 L/min flow rate using air or air containing 5% CO₂. AT-II is a high output, efficient nebulizer demonstrated to produce aerosols in the optimal range of 1-2 µm mass median aerodynamic diameter (MMAD) for peripheral lung delivery (17,18). The PEI-DNA aerosol particle size was calculated to be 1.6 µm MMAD with a geometric standard deviation (GSD) of 2.9 using Andersen Cascade Impactor (Andersen Instruments, Atlanta, GA) by a method previously described (18). A source of dry air (Aridyne 3500, Timeter, Lancaster, PA) is delivered to a Bird 3M gas blender (Palm Springs, CA) attached to an air compressor and a CO₂ tank. The resulting mixture of air and CO₂ is delivered to the nebulizer. The final concentration of 5% CO₂ in air was determined using a Fyrite solution (Bacharach, Pittsburgh, PA). The nebulization of 10 ml solution took approximately 30 minutes.

### EXAMPLE 24

### CAT assay

Mice were anaesthetized and sacrificed after each time point and the lungs and other tissues were harvested and immediately frozen. A CAT ELISA assay kit (Boehringer Mannheim Gmbh, Mannheim, Germany) was used for measuring in vivo expression. The tissues were homogenized in 700 µl CAT assay lysis buffer using a Wig-L-Bug bead homogenizer (Crescent Dental Mfg., Lyons, IL). After centrifuging the homogenates, 200 µl of the extract was used for the CAT ELISA assay performed in a 9 6 well plate format. The absorbance was read using a microtitre plate reader (Molecular Devices, Sunnyvale, CA). Naive mice were used as controls. The CAT activity is expressed as ng of CAT/ ml of tissue extract using a standard curve prepared with purified CAT enzyme. The sensitivity of the assay was further enhanced according to suggestions from the manufacturer so that it can detect levels of CAT protein as low as 0.1-0.3 pg/well.

### EXAMPLE 25

### Luciferase assay

Mice were anaesthetized and sacrificed and the lungs were harvested. A luciferase assay kit (Promega, Madison, WI) was used to measure luciferase expression. The lungs were homogenized in 1 ml of luciferase assay lysis buffer using a Wig-L-Bug bead homogenizer. After centrifuging the homogenates, 1 0 µl of the extract was added to 50 µl of luciferase substrate and the luminescence read for 10 seconds in a 96 well plate on a luminometer (Microlumat LB 96 P, EG&G Berthold, Germany). Naive mice were used as controls. The luciferase activity is expressed as RLU/10 sec/ml of extract. In this system, 10⁷ RLU corresponds to 1 ng of luciferase using purified luciferase from Promega.

### EXAMPLE 26

### Histotogical analysis of tissue sections

Mice were anaesthetized with isoflurane and sacrificed by exsanguination via the abdominal aorta. Lungs were isolated, cannulated and fixed by inflation with 10% neutral buffered formalin, embedded in paraffin and processed for histological analysis. Thin sections were cut at 4 µm and observed under the microscope to look for any signs of inflammation or toxicity using the hematoxylin and eosin stain. After performing one-way analysis of variance (ANOVA) to compare the means, a two-tailed unpaired Student's t-test was done. A difference was considered significant if p ≤ 0.05.

### EXAMPLE 27

### Nebulization of PEI-DNA complexes with 5% CO₂

Nebulization of PEI-DNA complexes with 5% CO₂ enhances the transgene expression in lung compared to normal air: Breathing 5% CO₂ in air has been associated with an increase in the tidal volume and breathing frequency in mice and humans (19,20,21). Inhalation of aerosols containing 5% CO₂ could lead to greater inhalation of aerosol particles and correspondingly higher transgene expression compared to that achieved with aerosol delivered by air. To investigate this, PEI-DNA complexes were delivered to Balb/C mice by aerosol using either normal air or air containing 5% CO₂. A fixed amount of CAT plasmid (1 m g / 10 ml of solution), at a N:P ratio of 10:1, was aerosolized for 30 minutes as indicated. The lungs were harvested after 24 hours and CAT assay was performed to determine the degree of transfection. 5% CO₂-in-air lead to a 3-fold increase (p = 0.001 ) in the levels of CAT detected compared to aerosol nebulized with air alone (Figure 9).

### EXAMPLE 28

### DNA transfer by PEI is dose dependent

To further optimize the transgene expression, the N:P ratio was kept constant at 10:1 and the amount of DNA was varied from 250 µg to 4 mg per 10 ml of the aerosolized solution. This leads to an increase in the reservoir concentration as well as the amount of total DNA nebulized in the aerosol output. The complexes were aerosolized using 5% CO₂-in-air with 2 mg DNA giving the highest level of CAT expression in the lung (Figure 10). The levels of CAT measured with 250 µg DNA were not statistically different from control lungs (p = 0.34). Also, when 4 mg of DNA was dissolved in 10 ml at N:P ratio of 10:1, it lead to some visual precipitation of the DNA which may account for no further increase in the level of CAT detected in the lungs as compared to 2 mg (p = 0.51).

### EXAMPLE 29

### Optimization of PEI-DNA ratios

To determine the charge ratio that would be ideal for in vivo aerosol delivery, different PEI-DNA (N:P) ratios were evaluated for their ability to transfect the lung. The amount of DNA was kept constant at 2 mg and the polyethyleneimine concentration was varied to obtain ratios of 5:1, 10:1, 12.5:1, 15:1, 17.5:1 and 20:1. These ratios were chosen based on previous in vitro and in vivo (by instillation) studies. The complexes were aerosolized using 5% CO₂-in-air. A N:P ratio of 15:1 gave highest level of CAT expression in lung whereas 5:1 resulted in a very low level of CAT expression (Figure 11). There was statistically no difference between 10:1, 12.5:1, 17.5:1 and 20:1 ratios (p > 0.1) but a significant difference between 15 :1 and 20:1 (p = 0.05) and between 10:1 and 15:1 (p = 0.014).

In order to determine the optimal ratio for a plasmid other than CAT, different N:P ratios were tested for the expression of luciferase gene in the lung. The ratios evaluated were 5:1, 10:1, 15:1, 20:1, 30:1 and 40:1. The optimum curve for luciferase shifted to the right as compared to CAT, with the highest expression at 20:1 (p < 0.05 as compared to other ratios) (Figure 12). This suggests that different plasmids might require different N:P ratios.

### EXAMPLE 30

### Time course of CAT expression following single aerosol delivery

The persistence of CAT expression following a single aerosol delivery was analyzed next. This is important information for planning a treatment regime for therapeutic studies. Two m g of CAT plasmid was aerosolized, using 5% CO₂-in-air, to the mice at two different N:P ratios- 15:1 and 10:1. Different time points examined for the 10:1 group were 1, 2, 3 and 6 days post aerosol exposure. Lungs and other tissues were harvested at different time points and frozen immediately. All tissues were assayed simultaneously after the last time point (day 6). For the 15:1 group the mice were sacrificed 1, 3, 7 and 10 days after aerosol treatment. The lungs were harvested and frozen after each time point and the CAT protein was assayed after the last time point (day 10).

For both N:P ratios examined, the CAT expression is highest at 24 hours and remains constant (statistically no difference between day 1 and day 3, p = 0.4 for 15:1 ratio and p = 0.12 for 10:1 ratio) for over 3 days (Figure 13A and 13B). The CAT level falls to about 50% of peak levels after a week and significant levels are detected even after 10 days (p = 0.001 compared to control).

### EXAMPLE 31

### Tissue distribution of transgene

Intravenous or intraperitoneal delivery of DNA vectors generally results in expression in a variety of tissues. To determine if aerosol delivery of PEI-DNA would also result in systemic gene delivery, different tissues were harvested from the same group of mice as the above experiment (from 10:1 group) and the CAT assay was performed after the last time point. The tissues examined were lung, liver, spleen, kidney, thymus, brain and blood. The level of CAT detected in non- lung tissues was very low and not significantly different (p > 0.1 for all the tissues) from the control tissues (Figure 14) indicating that aerosol delivery of PEI-DNA complexes results in highly specific pulmonary gene expression with no systemic delivery.

### EXAMPLE 32

### Histological analysis shows no signs of inflammation

To determine if aerosol delivery of PEI-DNA complexes would lead to any kind of toxicity or acute inflammation in this system, 2 mg of CAT plasmid was complexed with PEI at a N:P ratio of 15:1 and the mice were exposed to aerosol for 30 minutes using 5% CO₂-in-air The mice were sacrificed after 24 hours and the lungs were fixed in formalin and stained with hematoxylin and eosin. The lungs did not show any evidence of histological abnormality as shown in Figure 15. Use of 5% CO₂-in-air to optimize pulmonary gene delivery by PEI-DNA aerosol seems to be safe and highly specific for the lung.

### EXAMPLE 33

### PEI-Antitumor Gene Composition Effects in Animal Studies

Figures 15 and 16 shows the effects of PEI-antitumor gene compositions in animal studies. Figure 15 shows that compositions of PEI and the p53 gene and PEI and the retinoblastoma gene significantly reduced tumor growth in an animal model of melanoma when administered by aerosol twice weekly for 4 weeks. Figure 16 shows that compositions of PEI and the p53 gene and PEI, the p53 gene and the anticancer drug 9-nitrocamptothecin (9-NC) significantly reduced tumor growth in an animal model of adenocarcinoma when administered by aerosol twice weekly for 4 weeks. Figure 17 shows that compositions of PEI and the p53 gene significantly reduced tumor growth in a nude mouse model of human osteosarcoma lung metastasis (SAOS-LM6) when the composition was delivered by aerosol twice weekly for 4 weeks. Aerosol therapy was initiated 8 weeks after the animals had been implanted with the tumor cells. In this same experiment, PEI alone and PEI in combination with either the CAT or IL-12 genes (as nonspecific controls) did not result in inhibition significantly lower than the untreated control.

In this study, the use of 5% CO₂-in-air for nebulization of PEI-DNA leads to increased levels of gene expression in the lung over that achieved using conventional air during nebulization. This could be due to the increased tidal and minute volumes in the presence of 5% CO₂. The use of 5% CO₂ in aerosol results in a 4-5 fold greater deposition of drug-liposome particles in the lungs of rodents. When 5% CO₂-in-air was utilized to deliver the PEI-DNA aerosol, it could be observed visually that the mice were breathing deeper and more rapidly suggesting that increased transgene expression in lung could be due to increased tidal volume and breathing frequency and corresponding increase in deposition of aerosol particles. However it is possible that enhanced CO₂ has an effect on the transfection efficiency of PEI-DNA complexes by changing some other physiological parameters. Based on these observations, 5% CO₂-in-air could be used for aerosol delivery of other polymer-DNA or cationic lipid-DNA complexes and could give similar results. 5% CO₂ has been well tolerated by humans and shown to increase the minute volume, so this strategy could be potentially applied to patients with pulmonary diseases as well.

Other parameters for gene delivery by aerosol using PEI as a vehicle were also optimized. The charge interaction between any cationic vehicle and the negatively charged DNA is an important factor determining the efficiency of the transfection of the complex. Gene delivery by aerosol could require different conditions. A N:P ratio of 15:1 gave the highest levels of CAT expression in this system with 5:1 resulting in a very low level of expression as compared to other ratios.

In contrast, the highest expression for luciferase was obtained at a N:P ratio of 20:1. This could be due to the different size of luciferase plasmid leading to a structurally different complex with PEI as compared to CAT plasmid. It could also be due to a difference in plasmid purity and the proportion of supercoiled structure. Still there is a considerable overlap in the optimum N:P ratios of these two plasmids. The optimum ratios for different plasmids may be different. Considering experimental variability, a ratio between 10:1 and 20:1 should work suitably. A ratio lower than 10:1 did not give very high transfection in the lung.

The dose response of CAT expression shows that 2 mg of DNA complexed to PEI per 10 ml of aerosolized solution would give the best expression in this CO₂ enhanced delivery system. The expression sharply falls to insignificant levels when 250 µg of DNA/ 10 ml solution is nebulized to the mice. Four mg of DNA complexed to PEI (at various different ratios) lead to some precipitation of the DNA and this probably is the reason for decreased transfection. It should be noted that there is an increase in both the concentration and the amount of DNA delivered.

The nebulized output from the Aerotech II nebulizer was calculated to be approximately 80%. About 72% of the reservoir DNA was delivered to the inhalation chamber as estimated using an all-glass impinger (AGI) as previously described. The remainder was trapped in the T-connector and tubing. Based on murine obligate nasal breathing, pulmonary physiology (minute volume and deposition fraction), and the output concentration of aerosol (4.8 µg/L), the amount of DNA deposited in the lungs of a mouse is estimated to be approximately 4-5 µg during 30 minutes of aerosol exposure (for starting reservoir concentration of 2 mg DNA/10 ml solution). These calculations are based on normal air breathing and the deposition may be higher in the presence of 5% CO₂ due to the increased tidal volume and breathing frequency.

The CAT expression was also used to monitor the time course of gene expression. After a single aerosol exposure, CAT expression in lung is highest at 24 hours and then drops to about 50% of peak levels after a week. There was a very significant amount of expression (30% of peak levels) even after 10 days. This suggests that the persistence of gene expression following polyethyleneimine aerosol delivery may be more than adequate for a variety of clinical applications. The persistence of gene expression up to day 10 in the study is similar to or greater than that reported in other studies using cationic lipids for instillation or aerosol delivery of genes.

The tissue distribution data shows that gene expression following aerosol delivery in the system is confined to the lung indicating minimal systemic delivery. In contrast to the lung, tissues such as liver, spleen and kidney, that normally exhibit detectable levels of expression when genes are delivered via intravenous or intraperitoneal administration, exhibited insignificant or no detectable CAT expression when delivered by PEI-DNA aerosol. This is important if the expression of the gene of interest is to be restricted to the lungs. Aerosol delivery helps to distribute the particles non-invasively and uniformly throughout the lungs.

Lastly, but importantly, the lungs did not show any sign of inflammation. There was no inflammatory cell infiltration or damage to the lungs when thin sections were examined. Although high levels of expression are detected even after a week, some therapies might require repeated and frequent delivery of genes.

5% CO₂-in-air can increase the levels of gene expression when the gene is delivered by aerosol. The expression is predominant in the lung and highly significant levels are detected even after a week of aerosol delivery. PEI also appears to be non-toxic at the concentrations optimal for in vivo expression. Aerosol delivery of branched PEI-DNA complexes represents an inexpensive alternative to viral and cationic lipid vectors mediated gene delivery and should be useful for diseases like cystic fibrosis and lung cancer.

The following references were cited herein:
Boussif, O., et al. (1995) *Proc. Natl. Acad. Sci.* USA 92, 7297-7301.
Boussif, O., et al. (1996) *Gene Therapy* 3, 1074-1080.
Crook K., et al. (1996) *Gene Therapy* 3, 834-839.
Duncan, J.E., et al. (1997) *Human Gene Therapy* 8, 431-438.
Eastman S.J., et al. (1997) *Human Gene Therapy* 8, 765-773.
Eastman S.J., et al. (1998) *Human Gene Therapy* 9, 43-52.
Eastman SJ, et al. (1997) *Human Gene Therapy* 8, 313-322.
Gao, X., & Huang, L. (1991) *Biochem. Biophys. Res. Comm.* 179, 280-285.
Goula, D., et al. (1998) *Gene Therapy* 5, 1291-1295.
Haensler, J. & Szoka, F.C. (1993) *Bioconj Chem.* 4:372-379.
Kircheis, R., et al. (1997) *Gene Therapy* 4, 409-418.
Ogris, M., et al. (1999) *Gene Therapy* 6, 595-605.
Ogris, M., et al. (1998) *Gene Therapy* 5, 1425-1433.
Schwarz L.A., et al. (1996) *Human Gene Therapy* 7, 731-741.
Tang, M.X., & Szoka, F.C., (1997) *Gene Therapy* 4, 823-832.
Tsan, M.-F., et al. (1997) *Human Gene Therapy* 8, 817-825.
Vigneron, et al. (1996) *Proc. Natl. Acad. Sci.* 93, 9682-9686.
Wu, G.Y., & Wu, C.H., (1988) *Biochemistry* 27, 887-892.

Any patents or publications mentioned in this specification are indicative of the levels of those skilled in the art to which the invention pertains. Further, these patents and publications are incorporated by reference herein to the same extent as if each individual publication was specifically and individually indicated to be incorporated by reference.

One skilled in the art will appreciate readily that the present invention is well adapted to carry out the objects and obtain the ends and advantages mentioned, as well as those objects, ends and advantages inherent herein. The present examples, along with the methods, procedures, treatments, molecules, and specific compounds described herein are presently representative of preferred embodiments, are exemplary, and are not intended as limitations on the scope of the invention. Changes therein and other uses will occur to those skilled in the art which are encompassed within the spirit of the invention as defined by the scope of the claims.

## Claims

1. Use of a jet nebulized small particle aerosol composition comprising polyethyleneimine and a genetic macromolecule in the preparation of a medicament for the treatment of disorders of the respiratory tract or cancer.

2. Use according to claim 1, **characterised in that** said genetic macromolecule is selected from the group consisting of DNA, a therapeutic gene, RNA, catalytically active nucleic acids, ribozymes, antisense oligonucleotides and modified nucleic acids.

3. Use according to claim 1 or claim 2, **characterised in that** said genetic macromolecule is DNA encoding a therapeutic gene.

4. Use according to claim 3, **characterised in that** said therapeutic gene comprises operably linked elements necessary for expression.

5. Use according to claim 3 or claim 4, **characterised in that** said therapeutic gene is selected from the group consisting of a cystic fibrosis transmembrane conductance regulator gene, a gene encoding HSV thymidine kinase, a gene encoding a tumor suppressor, a gene encoding a cytokine, a gene encoding a tumor-specifc antigen, a gene encoding an infectious agent-specific antigen and a gene encoding a targeted antibody.

6. Use according to any one of claims 3 to 5, **characterised in that** the final concentration of said therapeutic gene in said polyethyleneimine is no greater than about 10 µg DNA/50 nmoles polyethyleneimine nitrogen/ml and no less than about 0.1 µg DNA/50 nmoles polyethyleneimine nitrogen/ml.

7. Use according to any one of the preceding claims, **characterised in that** said composition is formulated at concentrations of about 0.1 µg DNA/20 nmoles polyethyleneimine nitrogen/ml to about 10 µg DNA/150 nmoles polyethyleneimine nitrogen/ml.

8. Use according to any one of the preceding claims **characterised in that** the composition further comprises serum.

9. Use according to any one of the preceding claims, **characterised in that** said polyethyleneimine is covalently conjugated to a cell-specific ligand.

10. Use according to claim 9, wherein said celt-specific ligand is transferring.

11. Use according to any one of the previous claims wherein said composition is produced by a method comprising the steps of:
dissolving a genetic macromolecule in a solution;
dissolving polyetheleneimine in a solution;
complexing said dissolved genetic macromolecule and said dissolved polyethyleneimine, thereby producing a polyethyleneimine-genetic macromolecule composition; and
placing said polyethyleneimine-genetic macromolecule composition in a device capable of nebulization, thereby producing a polyethyleneimine-genetic macromolecule composition.

## Patentansprüche

1. Verwendung einer durch eine Düse vernebelten Aerosol-Zusammensetzung aus kleinen Teilchen, die Polyethylenimin und ein genetisches Makromolekül bei der Bereitstellung eines Medikaments zur Behandlung von Störungen des Respirationstrakts oder Krebs umfasst.

2. Verwendung nach Anspruch 1, **dadurch gekennzeichnet, dass** genanntes genetisches Makromolekül aus der Gruppe ausgewählt ist, die aus DNA, einem therapeutischen Gen, RNA, katalytisch aktiven Nukleinsäuren, Ribozymen, Antisense-Oligonukleotiden und modifizierten Nukleinsäuren besteht.

3. Verwendung nach Anspruch 1 oder Anspruch 2, **dadurch gekennzeichnet, dass** genanntes genetisches Makromolekül DNA darstellt, die ein therapeutisches Gen kodiert.

4. Verwendung nach Anspruch 3, **dadurch gekennzeichnet, dass** genanntes therapeutisches Gen operativ verknüpfte Elemente, die zur Exprimierung notwendig sind, umfasst.

5. Verwendung nach Anspruch 3 oder Anspruch 4, **dadurch gekennzeichnet, dass** genanntes therapeutisches Gen aus der Gruppe ausgewählt ist, die aus einem Cystic Fibrosis Transmembrane Conductance Regulator-Gen; einem Gen, das HSV Thymidinkinase kodiert; einem Gen, das einen Tumorsuppressor kodiert; einem Gen, das ein Cytokin kodiert; einem Gen, das ein tumorspezifisches Antigen kodiert; einem Gen, das ein infektiöses agensspezifisches Antigen kodiert und einem Gen, das einen gezielten Antikörper kodiert, besteht.

6. Verwendung der Zusammensetzung nach einem der Ansprüche 3 bis 5, **dadurch gekennzeichnet, dass** die Endkonzentration von genanntem therapeutischen Gen in genanntem Polyethylenimin nicht größer als etwa 10 µg DNA/50 nmol Polyethylenimin-Stickstoff/ml und nicht kleiner als etwa 0,1 µg DNA/50 nmol Polyethylenimin-Stickstoff/ml ist.

7. Verwendung nach einem der vorstehenden Ansprüche, **dadurch gekennzeichnet, dass** genannte Zusammensetzung bei Konzentrationen von etwa 0,1 µg DNA/20 nmol Polyethylenimin-Stickstoff/ml bis etwa 10 µg DNA/150 nmol Polyethylenimin-Stickstoff/ml formuliert ist.

8. Verwendung nach einem der vorstehenden Ansprüche, **dadurch gekennzeichnet, dass** die Zusammensetzung weiter Serum umfasst.

9. Verwendung nach einem der vorstehenden Ansprüche, **dadurch gekennzeichnet, dass** genanntes Polyethylenimin zu einem zellspezifischen Liganden kovalent konjugiert ist.

10. Verwendung nach Anspruch 9, wobei genannter zellspezifischer Ligand überträgt.

11. Verwendung nach einem der vorstehenden Ansprüche, wobei genannte Zusammensetzung durch ein Verfahren hergestellt ist, das folgende Schritte umfasst:
Auflösen eines genetischen Makromoleküls in einer Lösung;
Auflösen von Polyethylenimin in einer Lösung;
Komplexieren von genanntem aufgelösten genetischen Makromolekül und genanntem aufgelösten Polyethylenimin, **dadurch** Herstellen einer Zusammensetzung aus Polyethylenimin und genetischem Makromolekül; und
Einbringen von genannter Zusammensetzung aus Polyethylenimin und genetischem Makromolekül in eine Vorrichtung, die zur Zerstäubung geeignet ist, **dadurch** Herstellen einer Zusammensetzung aus Polyethylenimin und genetischem Makromolekül.

## Revendications

1. Composition aérosol de fines particules nébulisées par jet comprenant de la polyéthylèneimine et une macromolécule génétique dans la préparation d'un médicament pour le traitement de troubles des voies respiratoires ou du cancer.

2. Utilisation selon la revendication 1, **caractérisée en ce que** ladite macromolécule génétique est sélectionnée parmi le groupe constitué d'ADN, d'un gène thérapeutique, d'ARN, d'acides nucléiques actifs du point de vue catalytique, de ribozymes, d'oligonucléotides antisens et d'acides nucléiques modifiés.

3. Utilisation selon la revendication 1 ou la revendication 2, **caractérisée en ce que** ladite macromolécule génétique est l'ADN encodant un gène thérapeutique.

4. Utilisation selon la revendication 3, **caractérisée en ce que** ledit gène thérapeutique comprend des éléments liés de manière exploitable nécessaires pour l'expression.

5. Utilisation selon la revendication 3 ou la revendication 4, **caractérisée en ce que** ledit gène thérapeutique est sélectionné parmi le groupe constitué d'un gène régulateur de la conductance membranaire de la mucoviscidose, un gène encodant la HSV-thymidine kinase, un gène encodant un suppresseur de tumeur, un gène encodant une cytokine, un gène encodant un antigène spécifique à une tumeur, un gène encodant un antigène spécifique à un agent infectieux et un gène encodant un anticorps ciblé.

6. Utilisation de la composition selon l'une quelconque des revendications 3 à 5, **caractérisée en ce que** la concentration finale dudit gène thérapeutique dans ladite polyéthylèneimine est inférieure à environ 10 µg ADN/50 nmoles d'azote de polyéthylèneimine/ml et supérieure à environ 0,1 µg ADN/50 nmoles d'azote de polyéthylèneimine/ml.

7. Utilisation selon l'une quelconque des revendications précédentes, **caractérisée en ce que** ladite composition est formulée à des concentrations d'environ 0,1 µg ADN/20 nmoles d'azote de polyéthylèneimine/ml à environ 10 µg ADN/150 nmoles d'azote de polyéthylèneimine/ml.

8. Utilisation selon l'une quelconque des revendications précédentes, **caractérisée en ce que** la composition comprend en outre un sérum.

9. Utilisation selon l'une quelconque des revendications précédentes, **caractérisée en ce que** ladite polyéthylèneimine est conjuguée de manière covalente avec un ligand spécifique à une cellule.

10. Utilisation selon la revendication 9, dans laquelle ledit ligand spécifique à une cellule est transporteur.

11. Utilisation selon l'une quelconque des revendications précédentes, dans laquelle ladite composition est produite par un procédé comprenant les étapes consistant à :
dissoudre une macromolécule génétique dans une solution;
dissoudre une polyéthylèneimine dans une solution;
rendre complexe ladite macromolécule génétique dissoute et ladite polyéthylèneimine dissoute, produisant ainsi une composition de polyéthylèneimine et de macromolécule génétique; et
placer ladite composition de polyéthylèneimine et de macromolécule génétique dans un dispositif capable de nébulisation, produisant ainsi une composition de polyéthylèneimine et de macromolécule génétique.
